# EUROPEAN PATENT APPLICATION

(11) **EP 1 428 891 A1**
(43) Date of publication of application: **16.06.2004**
(21) Application number: 02762840.3
(22) Date of filing: 23.08.2002
(51) Int. Cl.: C12Q 1/68, C12N 15/52, C12Q 1/02, G01N 33/566, G01N 33/50, A61K 38/44, A61P 35/00

(54) **NOVEL GENE NEDL-1**

(30) Priority: 24.08.2001 JP 2001254974; 18.04.2002 JP 2002116753
(71) Applicant: HISAMITSU PHARMACEUTICAL CO. INC., Tosu-shi, Saga-ken 841-0017 (JP); Chiba-Prefecture, Chiba-shi, Chiba 260-8667 (JP)
(72) Inventor: NAKAGAWARA, Akira, Chiba Cancer Ctr Res. Institute, Chiba-shi, Chiba 260-0801 (JP); MIYAZAKI, Kou, Chiba-shi, Chiba 266-0031 (JP)
(74) Representative: Grünecker, Kinkeldey, Stockmair & Schwanhäusser Anwaltssozietät
(86) International application number: PCT/JP2002/008524
(87) International publication number: WO 2003/018842

(57) **Abstract**

Diagnostic agent or kit for the prognosis of neuroblastoma containing a nucleic acid probe or primer utilizing the nucleic acids derived from the NEDL-1 gene or the NEDL-1 protein, as well as method for diagnosing the prognosis of neuroblastoma.

## Description

### Technical Field

This invention relates to nucleic acids derived from genes expressed in neuroblastoma and gene expression products encoded by the nucleic acids. More particularly, the invention relates to nucleic acids and their fragments derived from the marker genes whose expression is enhanced in neuroblastomas with favorable prognosis based on comparison between neuroblastomas with favorable prognosis and neuroblastomas with unfavorable prognosis as well as to their utility in the diagnosis for the prognosis of neuroblastomas.

### Background Art

### (Tumorgenesis and Genes)

Individual tumors exhibit distinct characteristic natures, and their biological properties are not necessarily identical even though the basic principle of oncogenesis is the same. Rapid advances in the understanding of cancer from a molecular biological and molecular genetic perspective in recent years have opened the way to an explanation of oncogenesis and tumor cell biology on the genetic level.

### (Neuroblastomas)

Neuroblastoma is a pediatric cancer occurring in sympathetic gangliocytes and adrenal medullary cells which originate from cells of the peripheral sympathetic nervous system. Of these sympathetic nervous system cells, neural crest cells in the initial stage of development migrate to the abdomen, differentiating and maturing at sites where sympathetic ganglia are formed. Some of these cells migrate further to the adrenal bodies, penetrating through the adrenal cortex which is already in the process of formation, and reaching the medulla and forming medullary substance there. The neural crest cells also serve as a source of other peripheral nerve cells, differentiating into dorsal root ganglia (sensory nerves), skin pigment cells, thyroid C cells, some pulmonary cells, intestinal gangliocytes, and the like.

### (Prognosis of neuroblastoma)

Neuroblastoma is characterized by a varied clinical profile (Nakagawara, Shinkeigashu no Hassei to Sono Bunshi Kiko [Neuroblastoma Development and Molecular Mechanism], Shoni Naika 30, 143, 1998). For example, neuroblastoma occurring at less than one year of age has very favorable prognosis, with the majority undergoing differentiation and cell death, and spontaneous regression. Currently, most neuroblastomas discovered by a positive result in the commonly performed mass screening of 6-month-old infant urine are of the type which tend to undergo this spontaneous regression. On the other hand, neuroblastoma occurring at age 1 or higher is highly malignant and leads to death of the infant in the majority of cases. It is also hypothesized that a somatic mutation occurs in highly malignant neuroblastomas in infants older than one year of age, which are of monoclonal nature, whereas in naturally regressing neuroblastomas, the genetic mutation remains at only a germline mutation. See Knudson AG, et al.: Regression of neuroblastoma IV-S: A genetic hypothesis, N. Engl. J. Med. 302, 1254 (1980)).

### (Genes which allow the diagnosis for prognosis of neuroblastoma)

With recent advances in molecular biology research, it has become clear that expression of the high affinity nerve growth factor (NGF) receptor TrkA is closely connected with control of differentiation and cell death. See Nakagawara A., The NGF story and neuroblastoma, Med. Pediatr. Oncol., 31, 113 (1998). Trk is a membrane-spanning receptor, existing as the three main types, Trk-A, -B and -C.

These Trk family receptors play an important role in specific nerve cell differentiation and survival in the central nervous and peripheral nervous systems. See Nakagawara, et al., Shinkeigasaiboushu ni Okeru Neurotrophin Juyoutai no Hatsugen to Yogo [Expression of Neurotrophin Receptors and Prognosis in Neuroblastoma], Shoni Geka (Pediatric Surgery), 29: 425-432, 1997. The survival and differentiation of tumor cells is controlled by signals from Trk tyrosine kinase and Ret tyrosine kinase. In particular, the role of TrkA receptor is most significant, with TrkA expression being notably high in neuroblastomas with favorable prognosis, and its signals exerting a powerful control over survival and differentiation of tumor cells, and cell death (apoptosis). In neuroblastomas with unfavorable prognosis, on the other hand, TrkA expression is significantly suppressed, while tumor development is aided by a mechanism in which survival is promoted by signals from TrkB and Ret.

It has become clear that amplification of the neural oncogene N-myc has become clearly associated with the prognosis of neuroblastoma. See Nakagawara, Nou-shinkeishuyo no Tadankai Hatsugan [Multistage Oncogenesis of Cerebral and Neural Tumors], Molecular Medicine, 364, 366 (1999). This gene, first cloned in neuroblastoma, is ordinarily only present in a single copy per haploid set in normal cells and neuroblastomas with favorable prognosis, whereas it has been found to be amplified several dozen times in neuroblastomas with unfavorable prognosis.

Up till the present time, however, no oncogene other than N-myc is known to be expressed in neuroblastomas, and absolutely no genetic information other than that of N-myc has been known in relation to favorable or unfavorable prognosis.

### Disclosure of the Invention

This invention has been accomplished in light of these circumstances, and its object is to identify the base sequences of genes which are related to favorable or unfavorable prognosis of neuroblastoma, and to allow the diagnosis for the prognosis of neuroblastoma (whether favorable or unfavorable) based on their genetic information. Its object is also to provide the information on the functions of proteins which are the transcripts of the aforementioned genes.

As a result of conducting diligent research, the present inventors examined the prognoses of neuroblastomas and succeeded in constructing cDNA libraries from the respective clinical tissues with favorable prognosis and with unfavorable prognosis. Approximately 2400 clones were respectively obtained from these two types of cDNA libraries and were classified according to the prognosis of neuroblastomas and carried out profiling of the respective subsets.

Thus, the present inventors found that a group of genes showed differential expression levels among the abovementioned subsets and showed enhanced expression levels only in clinical tissues of neuroblastoma with favorable prognosis; one of the genes was designated "NEDL-1 (nbla0078)." Moreover, the present inventors sequenced the whole length of NEDL-1 gene, and conducted the functional analysis of NEDL-1 protein encoded by the gene: the protein was found to be a ubiquitin ligase of the HECT type.

Based on this finding the present inventors have made it possible to provide genetic information (base sequence data etc.) which allowed the detection and cloning of genes whose expression is enhanced only in the clinical tissues of neuroblastoma with favorable prognosis. Further based on the base sequence data, the present inventors made it possible to provide methods of diagnosis for prognosis and diagnostic agents therefor and thus completed this invention.

Specifically, this invention provides a nucleic acid probe comprising nucleic acid (a) or nucleic acid (b) described below:
(a) a nucleic acid having a portion of a base sequence set forth in SEQ ID NO:2 in the Sequence Listing or a base sequence complementary thereto; or
(b) a nucleic acid capable of hybridizing to the nucleic acid comprising a base sequence set forth in SEQ ID NO:2 in the Sequence Listing, or having a base sequence complementary to said base sequence.

Preferably, the nucleic acid is DNA in the nucleic acid probe described above.

Also preferably, the nucleic acid has a base length of at least 20 bases in the nucleic acid probe.

Further preferably, the base sequence set forth in SEQ ID NO:2 is its full-length in the nucleic acid probe.

This invention also provides a diagnostic agent for the prognosis of neuroblastoma comprising the nucleic acid probe described above as the effective ingredient.

This invention further provides a primer containing DNA (a) or DNA (b) as described below:
(a) DNA having a portion of a base sequence set forth in SEQ ID NO:2 in the Sequence Listing or a base sequence complementary thereto; or
(b) DNA capable of hybridizing to the DNA comprising a base sequence set forth in SEQ ID NO:2 in the Sequence Listing, or having a base sequence complementary to said base sequence.

This invention also provides a kit for the prognosis of neuroblastoma comprising the primer described above as the effective ingredient.

This invention further provides a method for diagnosing the prognosis of neuroblastoma, the method comprising detecting the presence or absence of a nucleic acid comprising a base sequence set forth in SEQ ID NO:2 in the Sequence Listing in a clinical tissue sample of neuroblastoma.

This invention also provides a method for diagnosing the prognosis of neuroblastoma, the method comprising detecting the presence or absence of a protein comprising an amino acid sequence set forth in SEQ ID NO:1 in the Sequence Listing in a clinical tissue sample of neuroblastoma.

This invention additionally provides a method for diagnosing the prognosis of neuroblastoma, the method comprising contacting with a clinical tissue sample of neuroblastoma, (a) a nucleic acid having a portion of a base sequence set forth in SEQ ID NO:2 in the Sequence Listing or a base sequence complementary thereto or (b) a nucleic acid capable of hybridizing to the nucleic acid comprising a base sequence set forth in SEQ ID NO:2 in the Sequence Listing, or having a base sequence complementary to said base sequence; and analyzing the expression of a protein comprising an amino acid sequence set forth in SEQ ID NO:1 in the Sequence Listing or a level thereof.

Accordingly, the nucleic acids and the proteins are derived from the marker genes whose expression is enhanced in neuroblastomas with favorable prognosis based on comparison between neuroblastomas with favorable prognosis and neuroblastomas with unfavorable prognosis. The information on the sequences of the nucleic acids and proteins will characteristically enable the diagnosis for the prognosis of neuroblastoma.

Further, this invention provides a polyubiqutination agent comprising as the effective ingredient, a protein comprising a base sequence set forth in SEQ ID NO: 1 in the Sequence Listing.

In the polyubiquitination agent, the substrate to be ubiquitinated is preferably β-amyloid precursor protein (βAPP), β-amyloid precursor protein intracellular region (AICD) or a superoxide dismutase mutant (SOD1).

This invention also provides a composition for modulating β-amyloid precursor protein (βAPP), the composition comprising an effective amount of a protein comprising an amino acid sequence set forth in SEQ ID NO:1 in the Sequence Listing to modulate the expression, the production or the formation of β-amyloid precursor protein in a cell.

This invention also provides a composition for modulating β-amyloid precursor protein (βAPP), the composition comprising an effective amount of a nucleic acid comprising a base sequence set forth in SEQ ID NO:2 in the Sequence Listing to modulate the expression, the production or the formation of β-amyloid precursor protein in a cell.

Further, this provides a method for modulating the expression, the production or the formation of β-amyloid precursor protein in a cell, the method comprising administering an effective amount of a protein comprising an amino acid sequence set forth in SEQ ID NO:1 in the Sequence Listing to modulate the expression, the production or the formation of β-amyloid precursor protein in a cell.

Still further, this provides a method for modulating the expression, the production or the formation of β-amyloid precursor protein in a cell, the method comprising administering an effective amount of a nucleic acid comprising a base sequence set forth in SEQ ID NO:2 in the Sequence Listing to modulate the expression, the production or the formation of β-amyloid precursor protein in a cell.

This invention also provides a composition for modulating superoxide dismutase (SOD1) activity, the composition comprising an effective amount of the protein comprising an amino acid sequence set forth in SEQ ID NO:1 in the Sequence Listing to modulate the superoxide dismutase (SOD1) activity in a cell.

This invention also provides a composition for modulating superoxide dismutase (SOD1) activity, the composition comprising an effective amount of a nucleic acid comprising a base sequence set forth in SEQ ID NO:1 in the Sequence Listing to modulate the superoxide dismutase (SOD1) activity in a cell.

This invention also provides a method for modulating superoxide dismutase (SOD1) activity in a cell, the method comprising administering an effective amount of a protein comprising an amino acid sequence set forth in SEQ ID NO:1 in the Sequence Listing to modulate the superoxide dismutase (SOD1) activity.

Further, this invention provides a method for modulating superoxide dismutase (SOD1) activity in a cell, the method comprising administering an effective amount of a nucleic acid comprising a base sequence set forth in SEQ ID NO:2 in the Sequence Listing to modulate the superoxide dismutase (SOD1) activity.

In the composition for modulating superoxide dismutase (SOD1) activity as well as in the method for modulating superoxide dismutase (SOD1) activity in a cell, SOD1 is preferably a mutant type thereof.

### Brief Description of the Drawings

Fig. 1A is a schematic representation of the protein structure of ubiquitin ligases of the HECT type showing that each has a HECT domain at its C-terminus, plural WW domains at its center and a C2 domain at its N-terminus.
Fig. 1B is an alignment diagram showing the homology analysis between the amino acid sequence of NEDL-1 protein and the amino acid sequence of NEDL-2 protein, where each of the domains is underlined or boxed and conservative amino acids are indicated by asterisks.
Fig. 2 is an electropherogram showing the results of determination of the expression levels of the NEDL-1 gene in clinical samples of neuroblastomas with favorable prognosis and with unfavorable prognosis by semi-quantitative PCR.
Fig. 3A is a figure corresponding to an electropherogram showing the results of determination of the expression levels of the NEDL-1 gene in normal human tissues by semi-quantitative PCR.
Fig. 3B is a figure corresponding to an electropherogram showing the results of determination of the expression levels of the NEDL-1 gene in various neuroblastoma cell lines by semi-quantitative PCR.
Fig. 4 is a figure representing autoradiography of different tissue expression of the NEDL-1 gene in normal human tissues as analyzed by Northern blot.
Fig. 5 is an immunoblotted electropherogram showing the ubiquitin ligase activity of the NEDL-1 protein.
Fig. 6A is a Western blot showing cellular localization of the NEDL-1 gene (Cos7 cell).
Fig. 6B is a Western blot showing cellular localization of the NEDL-1 gene (CHP134 cell).
Fig. 7 is an immunoblotted electropherogram showing the interaction between the NEDL-1 protein and ACID as obtained by immunoprecipitation with anti-NEDL-1 antibody and detection with anti-FLAG antibody.
Fig. 8 is an immunoblotted electropherogram showing the interaction between the NEDL-1 protein and ACID as obtained by immunoprecipitation with anti-FLAG antibody and detection with anti-NEDL-1 antibody.
Fig. 9A is an immunoblotted electropherogram showing the ubiquitination of βAPP and ACID by the NEDL-1 protein as obtained by immunoprecipitation with anti-HA antibody and detection with anti-ubiquitin antibody.
Fig. 9B is an immunoblotted electropherogram showing the ubiquitination of FLAG-ACID by the NEDL-1 protein as obtained by immunoprecipitation with anti-FLAG antibody and detection with anti-ubiquitin antibody.
Fig. 10 is an immunoblotted electropherogram showing the ubiquitination of βAPP and ACID by the NEDL-1 protein as obtained by immunoprecipitation with anti-HA antibody and detection with an antibody that recognizes ACID.
Fig. 11 is an immunoblotted electropherogram showing the interaction between the NEDL-1 protein and SOD1 mutants as obtained by immunoprecipitation with anti-NEDL-1 antibody and detection with anti-FLAG antibody.
Fig. 12 is an immunoblotted electropherogram showing the interaction between the NEDL-1 protein and SOD1 mutants as obtained by immunoprecipitation with anti-FLAG antibody and detection with anti-NEDL-1 antibody.
Fig. 13 is an immunoblotted electropherogram showing the ubiquitination of SOD1 and SOD1 mutants by the NEDL-1 protein.

### Best Mode for Carrying Out the Invention

The nucleic acids (which will be referred to as "the nucleic acid of this invention") derived from the gene which is highly expressed in neuroblastomas with favorable prognosis (which will be referred to as "the NEDL-1 gene of this invention" or simply as "the NEDL-1 gene") and the protein encoded by the gene will be described in detail by referring to the preferred embodiments of the invention.

As stated above, the nucleic acids of this invention are derived from the NEDL-1 gene of the invention and they make up the gene or are obtained from the gene by an *in vivo* or *in vitro* process. There are no limitations on the base lengths of the nucleic acids and here they will be referred to as the nucleic acids of the invention, which include nucleic acid fragments corresponding to parts of the gene. When the base lengths are short, they can be synthesized by chemical techniques. The term "nucleic acid(s)" as used in this specification refers to, for example, DNA or RNA, or polynucleotides derived therefrom which are active as DNA or RNA, and preferably refers to DNA and/or RNA. The particularly preferred nucleic acid has a base sequence that is identical with the human cDNA sequence disclosed in this specification or that is complementary to the sequence.

The term "hybridize under stringent conditions" as used in this specification means that two nucleic acid fragments hybridize to each other under the hybridization conditions described by Sambrook, J. et al. in "Expression of cloned genes in *E. coli*", Molecular Cloning: A Laboratory Manual (1989), Cold Spring Harbor Laboratory Press, New York, USA, 9.47-9.62 and 11.45-11.61.

More specifically, the "stringent conditions" refers to hybridization at approximately 45 °C, 6.0 x SSC, followed by washing at 50 °C, 2.0 x SSC. The stringency may be selected by choosing a salt concentration in the washing step from approximately 2.0 x SSC, 50 °C as low stringency to approximately 0.2 x SSC, 50 °C as high stringency. Also, the temperature in the washing step may be increased from room temperature, or approximately 22 °C as low stringency conditions, to approximately 65 °C as high stringency conditions.

The term "nucleic acid(s)" as used in this specification refers to an isolated nucleic acid(s) and to a nucleic acid or a polynucleotide containing substantially no cellular substances or culture medium, if prepared by recombinant DNA techniques, or containing substantially no precursor chemical substances or other chemical substances, if prepared by chemical synthesis.

The term "favorable prognosis" as used in this specification refers to a condition of neuroblastoma in which the tumor is localized or has become a regressing or benign sympathetic ganglion neoplasm, and is judged to have low malignancy based on N-myc or other tumor markers (TrkA, chromosomal aberration). According to a preferred embodiment of the invention, a favorable prognosis is a case of stage 1 or 2, with an onset age of less than one year and survival without recurrence for 5 or more years after surgery, and with no noted amplification of N-myc in the clinical tissue; however, there is no limitation to such specific cases. The term "unfavorable prognosis" as used in this specification refers to a condition of neuroblastoma in which progression of the tumor has been observed, and it is judged to have high malignancy based on N-myc or other tumor markers. According to a preferred embodiment of the invention, an unfavorable prognosis is a case of stage 4, with an onset age of greater than one year, death within 3 years after surgery and noted amplification of N-myc in the clinical tissue; however, there is no limitation to such specific cases.

Neuroblastoma is a tumor consisting of actual nerve cells, of which only two types of tumor are known in humans, and analysis of the genes expressed therein is expected to provide very useful knowledge for understanding the biology of nerve cells. Specifically, it is extremely difficult, and practically impossible, to obtain site-specific homogeneous tissue from the brain or peripheral nerves. On the other hand, a neuroblastoma consists of an almost homogeneous nerve cell population (though tumorized) derived from peripheral sympathetic nerve cells, and thus offers the high possibility of obtaining homogeneous expression of neuro-related genes. Furthermore, since neuroblastoma is a type of cancer, it will characteristically have many important genes expressed in the immature stage of neurogenesis.

Clinically and biologically, neuroblastoma can be neatly classified into favorable prognosis and unfavorable prognosis types. Cancer cells from neuroblastoma with favorable prognosis are characterized by having a very slow rate of proliferation, with spontaneous regression beginning at some point. Findings to date have confirmed that nerve cell differentiation and apoptosis (nerve cell death) occur in the spontaneous regression, and that the differentiation which occurs in the maturation stages of normal nerve cells and programmed cell death are phenomena very closely resembling each other. Consequently, it is highly probable that the analysis of genes expressed in such tumors will lead to obtaining important genetic information relating to nerve cell differentiation and apoptosis.

NEDL-1 gene from which the useful genetic information can be obtained and the NEDL-1 protein encoded by the gene are found in clinical tissues of human neuroblastomas with favorable prognosis. These gene and protein are provided with the characteristics described below.

The NEDL-1 gene of this invention is a gene having the full length of 6,200 bases (coding region of 4,755 bases) and its base sequence is shown in SEQ ID NO:2 in the Sequence Listing. NEDL-1 protein encoded by the gene comprises 1585 amino acids and its full length is shown in SEQ ID NO:1 in the Sequence Listing. The base sequence and the amino acid sequence have been registered with GeneBank (HYPERLINK http://www.ncbi.nlm.nih.gov.) as Accession No. AB048365.

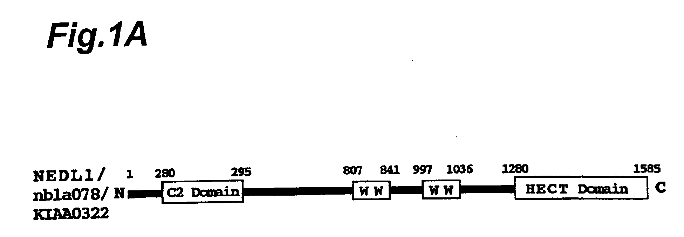

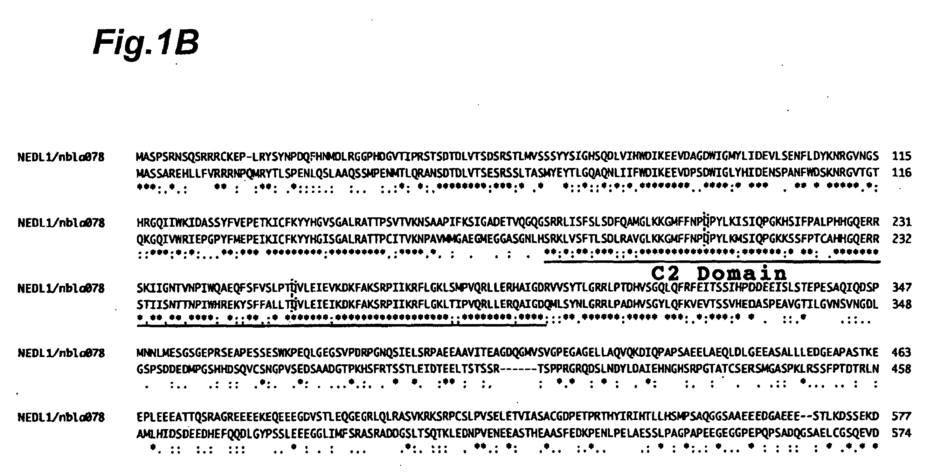

The present inventors found that as a result of the structural and functional analysis of the NEDL-1 gene and the NEDL-1 protein, NEDL-1 is a ubiquitin ligase of the HECT type. Fig. 1 shows the results from the homology analysis between the NEDL-1 protein and KIAA03222 protein (NEDL-2) which is a known member of the HECT type ubiquitin ligase family. The NEDL-1 protein has the domains characteristic of HECT type ubiquitin ligase. Specifically, these are (1) HECT domain (about 300 amino acids) at the C-terminus, which is positions 1280-1585 in NEDL-1; (2) plural WWW domains at the central part (about 35 to 40 amino acids), which is positions 807-841 and positions 998-1030 in NEDL-1 and positions 806-840 in NEDL-2; (3) C2 domain at the N-terminus binding to membrane lipid in a Ca-dependent manner, which is positions 185-295 in NEDL-1 and positions 186-295 in NEDL-2. In addition, the NEDL-1 protein was found to possess ubiquitin ligase activity at the same level as does Nedd4 which is a HECT type ubiquitin ligase.

Because proteins are decomposed in the ubiquitin-protease system, this system is essential to an adequate cellular process. In brief, the system allows a number of ubiquitin molecules (Ub) to be bound to a target protein (what is called "polyubiquitination or ubiquitination) and the ubiquitinated protein is decomposed by 26S proteasome. It has been elucidated that the ubiquitination of proteins progresses through the catalytic action of a series of enzyme groups, that is ubiquitin-activating enzymes (E1), ubiquitin-conjugating enzymes (E2), and ubiquitin-ligating enzymes (ubiquitin ligases). See, for example, a review of Keiji Tanaka, "Ubiquitin and Proteasome" in Experimental Medicine, Vol. 18, No. 11, pp. 1452-1456 (2000) by Yodosha. Among those enzymes ubiquitin ligase (E3) receives Ub from E2-Ub and ligate this Ub to the target protein (substrate). Thus, ubiquitin ligase is thought to be most heavily involved in the specificity with which a specific protein is ubiquitinated.

It has been pointed out that the anomaly in the ubiquitin-proteasome system is related to many diseases (R. J. Mayer et al., Biochem. Biophs. Acta 1089: 141-157 (1991)). Recently, the relation between neurodegenerative diseases and the anomaly in ubiquitin metabolism has attracted attention; and there has been a report that E6-AP, which is known as a ubiquitin ligase, is one of the responsible genes for Angelman syndrome (Nobutomi Honda et al., "HECT type ubiquitin-ligating enzymes: physiological functions and disease state" in Experimental Medicine, Vol. 18, No. 11, pp. 1483-1490 (2000) by Yodosha). The NEDL-1 protein of this invention, which is one of HECT type ubiquitin ligases, is highly expressed in nerve tissues. It is, therefore, well anticipated that the protein uses as a substrate the product of a causative gene of a neurodegenerative disease. These causative gene products are believed to be beta amyloid precursor protein (βAPP), preselinin protein (PS) and others.

As will be described in the Examples, it was actually found that NEDL-1 interacted with amyloid beta precursor intracellular domain (AICD) which was the coding region of amyloid precursor protein. It was further determined that this interaction resulted from ubiquitination of BAPP and ACID by NEDL-1.

Amyloid is a protein that deposits in cerebral blood vessels and senile plaques of an Alzheimer patient: it is comprised principally of β-protein with a molecular weight of 4 kDa and is produced when amyloid precursor protein is cleaved by secrease. The fact that NEDL-1 directly interacts with ACID leads to the possibility that NEDL-1 regulates the production of βAPP (then β amyloid) directly or indirectly. It will be a finding that is extremely important to planning a strategy for Alzheimer treatment which targets lowered production of β-amyloid on a molecular level.

As will also be described in the Examples, it was also found that NEDL-1 interacted with superoxide dismutase mutants (SOD1). It was further determined that this resulted from ubiquitination of the SOD1 mutants by NEDL-1.

Amyotrophic lateral sclerosis (ALS) is a neurodegenerative diseases with unfavorable prognosis that involves muscular atrophy due to the degeneration or deciduation of motor neurons. Currently, familial ALS is seen in a frequency of 5-10% of the total ALS. The causative gene has been identified as the Cu/Zn superoxide dismutase (SOD1) in some of the families. SOD1 is an enzyme that inactivates superoxide dismutase which is one type of active enzymes produced in a cell during an aerobic process. There is the possibility that its lowered level causes degeneration of nerve cells; however the detail of mechanism is unknown. The cause for other general type of amyotrophic lateral sclerosis is also unknown.

Presently, the virus theory, the poisoning theory, the nerve nutrient factor depletion theory, the autoimmune theory, the excessive glutamate theory, the free radical theory and others are proposed. However, the decisive pathological mechanism by which only motor nerve degenerates in ALS has not been elucidated. The aggregate hypothesis assumes that mutant SOD1 forms an aggregate in a cell and exhibits cytotoxity; and this will be becoming the most convincing one in recent years.

Thus far about 80 of SOD1 mutants have been reported. Intracellular signal transduction remain unknown for any of these mutants. There has been a report on interacting molecules for two types of SOD1 mutants (G85R/G93A) and only two types, lysyl-tRNA synthetase and translocon-associated protein delta, have been identified as a protein factor that only binds to those mutants and that does not bind to normal SOD1. (Kunst CB, Mezey E, Brownstein MJ, Patterson D, "Mutations in SOD1 associated with amyotrophic lateral sclerosis cause novel protein interactions" Nat. Genet. 1997 Jan; 15(1) 91-4.) The details have not yet been elucidated. Thus, about 130 years have passed since the first report of ALS, but even now the situation may be that the elucidation of the intracellular signal transduction for cytotoxity which the mutant SOD1 has newly acquired. In consideration of the present circumstance described above, the results obtained from this invention are believed to provide very useful information for the elucidation of the mechanism of ALS crisis that has not been hitherto clarified.

The NEDL-1 protein has an amino acid sequence set forth in SEQ ID NO:1 in the Sequence Listing but this invention also encompasses a protein having an amino acid sequence comprising a deletion, a substitution, an insertion or an addition of one or more amino acids in the amino acid sequence set forth in SEQ ID NO:1 in the Sequence Listing.

This invention also encompasses salts of the NEDL-1 protein and others. These salts are not particularly limited and, for example, preferred are a sodium salt, a potassium salt, a magnesium salt, a lithium salt and an ammonium salt.

Sugar chains are added to many proteins and the addition of a sugar chain may be adjusted by converting one or more amino acids. Therefore, this invention encompasses proteins the sugar chain addition of which has been adjusted in the amino acid sequence set forth in SEQ NO:1 in the Sequence Listing.

This invention further encompasses a nucleic acid having a base sequence encoding the NEDL-1 protein. The term "encoding a protein" as used herein means that either of complementary double strands has a base sequence encoding the protein when DNA is doublestranded. The nucleic acids of this invention embrace a nucleic acid comprising a base sequence directly encoding the amino acid sequence set forth in SEQ ID NO:1 in the Sequence Listing and a nucleic acid comprising a base sequence complementary to said nucleic acid.

Further, the nucleic acid of the invention may be a nucleic acid hybridizing to the nucleic acid comprising a base sequence set forth in SEQ ID NO:2 under stringent conditions. The base sequence is not particularly limited insofar as it satisfies this condition. Still further, the nucleic acids of the invention encompass a nucleic acid comprising a base sequence complementary to the nucleic acid hybridizable under the stringent conditions mentioned above. Specifically there is mentioned a nucleic acid comprising deletions, substitutions, insertions or additions in some bases of the nucleic acid comprising a base sequence set forth in SEQ ID NO:2 or a nucleic acid complementary to said nucleic acid. As used herein, the deletion, the substitution, the insertion and the addition include not only a short deletion, substitution, insertion and addition with 1 to 10 bases, but also a long deletion, substitution, insertion and addition with 10 to 100 bases.

As a result of comparing levels of expression of the NED-1 gene according to this invention in clinical tissues from neuroblastomas with favorable prognosis and with unfavorable prognosis, a highly significant difference was found. That is, expression of this gene was enhanced in neuroblastomas with favorable prognosis. Thus, in addition to providing the useful genetic information described above, the nucleic acid sequence set forth in SEQ ID NO:2 can also be utilized as data for tumor markers to diagnose favorable or unfavorable prognosis of neuroblastoma, by detecting the nucleic acid (DNA or RNA) having that sequence. The amino acid sequence in SEQ ID NO:1 can also be utilized as data for tumor markers to diagnose favorable or unfavorable prognosis of neuroblastoma, by detecting the NEDL-1 protein based on the sequence information.

Specifically, by using the NEDL-1 gene and the NEDL-1 protein according to this invention, the invention will make it possible to obtain various genetic information on or relating to neuroblastoma through the following means.

### (1) Probes for use in hybridization

According to one embodiment of this invention, the nucleic acid of the invention can be used as a probe (i.e., the probe of this invention) for hybridization to detect the NEDL-1 gene expressed in neuroblastoma. The nucleic acid according to this invention can also be used as probes for hybridization in order to determine gene expression in a variety of tumors and normal tissues, to identify the distribution of the gene expression.

When the nucleic acid according to this invention is used as a probe for hybridization, there are no particular limitations on the actual method of hybridization. As preferred methods there may be mentioned, for example, Northern hybridization, Southern hybridization, colony hybridization, dot hybridization, fluorescence *in situ* hybridization (FISH), *in situ* hybridization (ISH), DNA chip methods, and microarray methods.

As one application example of the hybridization, the nucleic acid according to this invention can be used as a probe for Northern hybridization to measure the length of mRNA or to quantitatively detect the expression of the NEDL-1 gene of this invention in a clinical tissue sample to be assayed.

As another application example, the nucleic acid according to this invention can be used as a probe for Southern hybridization to detect the presence or absence of the DNA sequence in the genomic DNA of a clinical tissue sample to be assayed.

As still another application example, the nucleic acid according to this invention can also be used as a probe for fluorescence in situ hybridization (FISH) to identify the location of the NEDL-1 gene of this invention on a chromosome.

As a further application example, the nucleic acid according to this invention can also be used as a probe for in situ hybridization (ISH) to identify the tissue distribution of expression of the NEDL-1 gene of this invention.

When the nucleic acid according to this invention is used as a probe for hybridization, a base length of at least 20 is necessary; and among the nucleic acids according to this invention, a nucleic acid having 20 or more contiguous bases is preferably used. More preferably, the nucleic acid having 40 or more bases is used and most preferably the nucleic acid having 60 or more bases is used. Further, the nucleic acid having the full-length of the base sequence set forth in SEQ ID NO:2 may be used.

Nucleic acid probe techniques are well known to one skilled in the art, and for example, conditions suitable for hybridization between a probe of specific length according to the invention and the target polynucleotide may be readily determined. In order to obtain hybridization conditions optimal to probes of varying lengths, Sambrook et al. "Molecular Cloning: A Laboratory Manual, 2nd Edition, Cold Spring Harbor (1989) may be followed for such manipulations which are well known to one skilled in the art.

The probe according to this invention may preferably be labeled for use in an easily detectable fashion. The detectable label may be any type and any element or compound which can be detected either visually or using devices. As commonly used detectable labels, there may be mentioned radioactive isotopes, avidin and biotin and fluorescent substances (FITC or Rhodamins). The radioactive isotopes are ³²P, ¹⁴C, ¹²⁵I, ³H, ³⁵S etc. Biotin-labeled nucleotides may be incorporated into DNA or RNA by nick translation, or chemical or enzymatic means. The biotin-labeled probes are detected after hybridization using labeling means such as avidin/streptavidin, fluorescent labels, enzymes, gold colloidal complexes or the like. The nucleic acid probe of this invention may also be labeled by binding with a protein. For this purpose, a radioactive or fluorescent histone single-stranded binding protein may also be used. In this manner, a suitably labeled probe constitutes a diagnostic agent for prognosis according to this invention.

### (2) Primers for use in PCR

For methods of detecting the NEDL-1 gene according to this invention other than the hybridization, primers can be designed after any nucleic acid (DNA) sequence contained in the nucleic acid according to this invention and the polymerase chain reaction (PCR) method can be used. For example, RNA may be extracted from a clinical tissue sample to be assayed, and the gene expression can be semi-quantitatively measured by RT-PCR. This may be carried out by a method well known to one skilled in the art. For example, "Molecular Cloning: A Laboratory Manual," (T. Maniatis, Cold Spring Harbor Laboratory Press) or Idenshibyo Nyumon [Introduction to Genetic Diseases] (Takahisa, S.: Nankodo Publishing) may be followed.

When the nucleic acid according to this invention (DNA) is used as a PCR primer (i.e., the primer of the invention), a base length of 10 to 60 is necessary; and among portions of the base sequences according to the invention, the nucleic acid having 10 to 60 contiguous bases is preferably used. More preferably, one having 15 to 30 bases is used. Generally, a primer sequence with a GC content of 40-60% is preferred. Also, there is preferably no difference in the Tm values of the two primers used for amplification. The primer has such base sequence that there is no annealing at the 3' ends of the primers and no secondary structure is formed in the primers.

### (3) Gene screening

The nucleic acid according to this invention can also be used to detect the expression distribution of the NEDL-1 gene which is expressed in various tissues or cells. This can be accomplished, for example, by using the nucleic acid according to this invention as a probe for hybridization or as a primer for PCR.

The expression distribution of the gene can also be detected using a DNA chip, microarray or the like. That is, the nucleic acid according to the invention may be directly attached to the chip or array. There is known a method by which nucleic acids (DNA) are spotted to a substrate for the purpose of attaching them to a chip or array by using a high precision dispenser (for example, see United State Patent No. 5807522). mRNA extracted from a clinical tissue sample may be labeled with a fluorescent substance or the like, hybridized thereto, and an analysis can be made of the type of tissue cells with high expression of the gene. The DNA attached to the chip or the array may be the reaction product of PCR using the nucleic acid or its fragment according to the invention. As an alternative method, the nucleic acid fragment of this invention (DNA fragment) may be directly synthesized on a substrate to form a DNA chip or a DNA array (See, for example, United State Patent No. 5424186).

### (5) Methods of diagnosing tumor prognosis and tumor markers to be used therefor

As mentioned above, the NEDL-1 gene of this invention has its expression enhanced in neuroblastomas with favorable prognosis. Therefore, the nucleic acid according to this invention can be used as a probe for hybridization, or as a primer for PCR to determine the presence or absence of enhancement in the gene expression in a sample containing the clinical tissue taken from the subject, which enables the identification of prognosis. The methods of detecting the gene include Northern blot hybridization, *in situ* hybridization and RT-PCR, as mentioned above among others.

When hybridization is employed, prognosis may be diagnosed as favorable if the amount of nucleic acid hybridizing to the probe is increased in the sample. When RT-PCR is employed, mRNA is extracted from the sample and reverse transcribed into DNA, amplification is performed using the aforementioned primer, and the gene expression is semi-quantitatively measured. The prognosis may be diagnosed as favorable if the gene expression is then found to be enhanced. For the purpose of such specific diagnosis it is preferred to utilize a diagnosis kit containing a pair of such primers as essential components. In addition to the primer components, the diagnosis kit also include known components such as PCR buffer, detergent solution and enzymes.

### (6) Antisense oligonucleotides

According to another embodiment of this invention there are provided antisense oligonucleotides to the nucleic acids of the invention. The antisense oligonucleotides are capable of hybridizing to the nucleic acids of the invention, and include antisense DNAs and antisense RNAs. Antisense DNA inhibits transcription of mRNA from DNA, while antisense RNA inhibits translation of mRNA. These antisense oligonucleotides may be synthesized using an automated synthesizer or by PCR using the nucleic acid of the invention as templates. The antisense oligonucleotides also encompass antisense oligonucleotide derivatives having improved binding affinity for DNA or mRNA, tissue selectivity, cell permeability, nuclease resistance and intracellular stability. These derivatives may be synthesized using antisense technology known in the art.

Antisense oligonucleotides having sequences complementary to the sequences near the translation initiation codon of the mRNA, those of the ribosome-binding site, and those of the capping site or the splicing site are capable of inhibiting synthesis of the RNA and therefore will exhibit a particularly notable inhibitory effect on gene expression. This invention therefore encompasses such antisense oligonucleotides.

### (7) Gene therapy

According to a further embodiment of this invention, there are provided nucleic acid sequences encoding the therapeutic genes to be used in gene therapy. Thus, the nucleic acid of the invention can be transferred into a vector for use in gene transportation, whereby the transgene (i.e., the NEDL-1 gene of the invention) can be expressed by an arbitrary expression promoter and can be used in the gene therapy for neurodegenerative diseases, for example.

### 1. Vectors

The transferable viral vectors may be prepared from DNA viruses or RNA viruses. They may be any viral vector of an MoMLV vector, a herpes virus vector, an Adenovirus vector, an AAV vector, a HIV vector, a SIV vector, a Seidai virus vector and the like. One or more proteins among the constituent protein group of a viral vector are substituted by the constituent proteins of a different species of virus, or alternatively a part of the nucleic acid sequence constituting genetic information is substituted by the nucleic acid sequence of a different species of virus to form a viral vector of the pseudo-type which can also be used in this invention. For example, there is mentioned a pseudo-type viral vector wherein the Env protein (an envelop protein of HIV) is substituted by the VSV-G protein (an envelop protein of vesicular stomatitis virus or VSV) (Naldini L., et al., Science 272, 263- 1996). Further, viruses having a host spectrum other than human are usable as the viral vector insofar as they are efficacious. As for the vectors other than those of viral origin, there may be used complexes of calcium phosphate and nucleic acid, ribosomes, cation-lipid complexes, Seidai virus liposomes, polymer carriers having polycation as the backbone main chain and others. In addition, methods such as electroporation and gene guns may be used as a gene transfer system.

### 2. Expression promoters

As for the expression cassettes to be used for the therapeutic gene, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, and most preferably expression cassettes capable of gene expression in the cells derived from a human. The gene promoters that can be used as expression cassettes include: for example, virus-derived promoters from an Adenovirus, a cytomegalovirus, a human immunodeficiency virus, a simian virus 40, a Rous sarcoma virus, a herpes simplex virus, a murine leukemia virus, a sinbis virus, a hepatitis type A virus, a hepatitis type B virus, a hepatitis type C virus, a papilloma virus, a human T cell leukemia virus, an influenza virus, a Japanese encephalitis virus, a JC virus, parbovirus B19, a poliovirus, and the like; mammal-derived promoters such as albumin, SRα, a heat shock protein, and an elongation factor; chimera type promoters such as a CAG promoter; and the promoters whose expression can be induced by tetracyclines, steroids and the like.

### (8) Drugs

According to a still further embodiment of this invention, there are provided therapeutic proteins and peptides as drugs. As will be considered in practicing this invention, the NEDL-1 protein of the invention and its partial peptide may be prepared according to the formulation method of choice and may be used through any desired route of administration and at any desired dosage age in the treatment of malignant tumors or neurodegenerative diseases (e.g., Alzheimer disease) of different types, for example.

### 1. Preparation method

The drug may be prepared as a recombinant viral vector containing a therapeutic gene that is designed for therapeutic purposes as described above. More specifically, a recombinant virus vector comprising the NEDL-1 gene may be prepared by dissolving it in an appropriate solvent such as water, physiological saline or an isotonized buffer solution. Alternatively, the NEDL-1 protein produced by any desired method may be dissolved in an appropriate solvent such as water, physiological saline or an isotonized buffer solution to prepare the vector similarly. Here, polyethylene glycol, glucose, various amino acids, collagen, albumin or the like may be then added as protective materials for the preparation.

### 2. Administration method and dosage

There are no particular limitations on the method of administrating the drug mentioned above to the living body. For example, parental administration, including injection is preferably carried out. The use level of the drug varies depending on the method of use, the purpose of use, etc.; and one skilled in the art can easily select as appropriate and optimize it. In the case of injection, for example, the daily dosage is preferably administered at about 0.1 µg/kg to 1000 mg/kg per day, and more preferably at about 1 µg/kg to 100 mg/kg per day.

### (9) Antibodies, Antisense, Ribozymes and TFO

In accordance with a still another embodiment of this invention, an antibody to suppress the ubiquitin activity of the NEDL-1 protein of the invention and base sequences, including antisense, ribozyme or TFO, to suppress the expression of the NEDL-1 gene of the invention are provided. As will be considered in practicing this invention, nucleic acids encoding antisenses, ribozymes and TFOs can be transferred into a vector used as a gene carrier; the transgene can be expressed by any suitable expression promoter and can be used, for example, to establish a primary culture cell line or to construct a cancer model animal.

### (10) Genetically modified animals

In accordance with a yet another embodiment of this invention, a nucleic acid sequence to knock out the expression of the NEDL-1 gene of the invention and a knockout animal (e.g., knockout mouse) are provided. There are provided a transgenic animal (e.g., transgenic mouse) where the gene has been forcedly expressed and a genetically modified animal having an introduced mutant gene obtained by introducing an arbitrary mutation (such as a point mutation or deletion) into the gene. This genetically modified animal can be used to construct a model animal for a neurodegenerative disease, for example.

As described above, by utilizing the NEDL-1 gene or the NEDL-1 protein according to this invention or the information obtainable therefrom, it will be possible to detect the NEDL-1 gene in a clinical tissue sample, which then will allow the diagnosis of neuroblastoma whether favorable or unfavorable prognosis. Further, by utilizing the gene, the protein or the information obtainable therefrom, it will be possible to design tumor markers that can be used in the diagnosis for prognosis and the aforementioned method.

This invention will now be explained in greater detail by way of the examples; however, the technical scope of invention will not be restricted to those examples.

### (Examples)

### (Preparation Example 1) Construction of cDNA library from neuroblastoma

### 1. Obtaining samples

The clinical tissue samples of neuroblastoma were quasi-aseptically frozen immediately after surgical extraction and then preserved at -80 °C.

### 2. Selecting samples with favorable prognosis

Prognosis of the samples obtained in 1. above was carried out based on the following criteria.

| Favorable prognosis | Unfavorable prognosis |
|---|---|
| · Stage 1 or 2 | · Stage 4 |
| · Age of onset: <1 | · Age of onset: ≥1 |
| · Survival for ≥5 years after surgery without recurrence | · Death within 3 years after surgery |
| · No amplification of N-myc | · Amplification of N-myc |

Amplification of N-myc in the aforementioned two sample types was confirmed in the following manner.

The samples obtained in 1. above was thinly sliced with a scalpel and then thoroughly homogenized after addition of 5 ml of TEN buffer (50 mM Tris-HCl (pH = 8.0)/1 mM EDTA/100 mM NaCl). Upon adding 750 µl of SDS (10%) and 125 µl of proteinase K (20 mg/ml) to the mixture, it was gently stirred and allowed to stand at 50 °C for 8 hours. This was followed by phenol/chloroform treatment and finally ethanol precipitation to obtain purified genomic DNA. A 5 µg portion of the obtained genomic DNA was completely digested with the restriction endonuclease EcoRI (NEB Inc.), and an N-myc probe was used to determine amplification of N-myc by Southern hybridization.

### 3. Preparation of mRNA from clinical tissue of neuroblastoma with favorable prognosis

A 2-3 g portion of the clinical tissue samples of neuroblastoma judged to have favorable prognosis in 2. above was treated using a Total RNA Extraction Kit (QIAGEN Inc.) and the total RNA was extracted. The extracted total RNA was purified using an oligo dT cellulose column (Collaborative Research, Inc.) to obtain a pool of mRNA with a polyA structure.

### 4. Dephosphorylation of mRNA

A 100-200 µg portion of the mRNA pool prepared in 3. above was dissolved in 67.3 µl of distilled sterile water containing 0.1% diethyl pyrocarbonate (DEPC), and then 20 µl of 5XBAP buffer (Tris-HCl (500 mM, pH = 7.0)/mercaptoethanol (50 mM)), 2.7 µl of RNasin (40 unit/µl: Promega Inc.) and 10 µl of BAP (0.25 unit/µl, bacteria-derived alkali phosphatase: Takara Shuzo Co. Ltd.) were added. The mixture was reacted at 37 °C for 1 hour to effect dephosphorylation of the 5' end of the mRNA. This was followed by phenol/chloroform treatment two times, and finally ethanol precipitation to obtain a purified dephosphorylated mRNA pool.

### 5. Decapping of dephosphorylated mRNA

The total amount of the dephosphorylated mRNA pool prepared in 4. above was dissolved in 75.3 µl of distilled sterile water containing 0.1% DEPC, and then 20 µl of 5XTAP buffer (sodium acetate (250 mM, pH = 5.5)/mercaptoethanol (50 mM), EDTA (5 mM, pH = 8.0)), 2.7 µl of RNasin (40 unit/µl) and 2 µl of TAP (tobacco acid pyrophosphatase: 20 unit/µl) were added. The mixture was reacted at 37 °C for 1 hour to effect decapping treatment of the 5' end of the dephosphorylated mRNA. The dephosphorylated mRNA of incomplete length with no capped structure remained without decapping, and with the 5' end dephosphorylated. This was followed by phenol/chloroform treatment and ethanol precipitation to obtain a purified decapped mRNA pool.

### 6. Preparation of oligo-capped mRNA

The total amount of the decapped mRNA pool prepared in 5. above was dissolved in 11 µl of distilled sterile water containing 0.1% DEPC, and then 4 µl of 5'-oligo RNA (5'-AGCAUCGAGUCGGCCUUGGCCUACUGG-3': 100 ng/µl), 10 µl of 10X ligation buffer (Tris-HCl (500 mM, pH = 7.0)/mercaptoethanol (100 mM)), 10 µ l of magnesium chloride (50 mM), 2.5 µl of ATP (24 mM), 2.5µl of RNasin (40 unit/µl), 10 µl of T4 RNA ligase (25 unit/µl: Takara Shuzo Co. Ltd.) and 50 µl of polyethylene glycol (50% w/v, PEG8000: Sigma Corporation) were added. The mixture was reacted at 20 °C for 3 hours for ligation of the 5'-oligo RNA to the 5' end of the decapped mRNA. The dephosphorylated mRNA of incomplete length with no capped structure resulted in no ligation to the 5'-oligo RNA. This was followed by phenol/chloroform treatment and ethanol precipitation to obtain a purified oligo-capped mRNA pool.

### 7. Removal of DNA from oligo-capped mRNA

The oligo-capped mRNA pool prepared in 6. above was dissolved in 70.3 µl of distilled sterile water containing 0.1% DEPC, and then 4 µl of Tris-HCl (1 M, pH = 7.0), 5.0 µl of DTT (0.1 M), 16 µl of magnesium chloride (50 mM) , 2.7 µl of RNasin (40 unit/µl) and 2 µl of DNaseI (5 unit/µl: Takara Shuzo Co. Ltd.) were added. The mixture was reacted at 37 °C for 10 minutes to dissolve the excess DNA. This was followed by phenol/chloroform treatment and ethanol precipitation and column purification (S-400HR: Pharmacia Biotech Inc.), to obtain a purified DNA(-) oligo-capped mRNA pool.

### 8. Preparation of 1st strand cDNA

The DNA(-) oligo-capped mRNA pool prepared in 7. above was reverse transcribed using SuperScript II (kit by Life Tech Oriental, Inc.) to obtain a pool of 1st strand cDNA. The pool of DNA(-) oligo-capped mRNA was dissolved in 21 µl of sterile distilled water, and then 10 µl of 10X First Strand buffer (kit accessory), 8 µl of dNTP mix (5 mM, kit accessory), 6 µl of DTT (0.1 M, kit accessory), 2.5 µl of oligo-dT adapter primer (5 pmol/ µ 1, 5'-GCGGCTGAAGACGGCCTATGTGGCCTTTTTTTTTTTTTTTTT-3'), 2.0 µl of RNasin (40 unit/µl) and 2 µl of SuperScript II RTase (kit accessory) were added. The mixture was reacted at 42 °C for 3 hours to effect reverse transcription. This was followed by phenol/chloroform treatment, alkali treatment and neutralization treatment to dissolve all the RNA and purification was carried out by ethanol precipitation.

### 9. Preparation of 2nd strand cDNA

The 1st strand cDNA pool prepared in 8. above was subjected to PCR amplification using Gene Amp (kit by Perkin Elmer Inc.). The pool of 1st strand cDNA was dissolved in 52.4 µl of sterile distilled water, and then 30 µl of 3.3X Reaction buffer (kit accessory), 8 µl of dNTP mix (2.5 mM, kit accessory), 4.4 µl of magnesium acetate (25 mM, kit accessory), 1.6 µl of Primer F (10 pmol/µ1, 5'-AGCATCGAGTCGGCCTTGTTG-3'), 1.6 µl of Primer R (10 pmol/ µ l, 5'-GCGCTGAAGACGGCCTATGT-3') and 2 µl of rTth (kit accessory) were added. A 100µl portion of mineral oil was gently added to the mixture and overlayed thereon. After denaturing the reaction solution at 94 °C for 5 minutes, a cycle of 94 °C for 1 minute, 52 °C for 1 minute and 72 °C for 10 minutes was repeated 12 times, and then the solution was allowed to stand at 72 °C for 10 minutes to complete the PCR reaction. This was followed by phenol/chloroform treatment and ethanol precipitation to obtain a 2nd strand cDNA pool.

### 10. SfiI treatment of 2nd strand cDNA

The 2nd strand cDNA pool prepared in 9. above was dissolved in 87 µl of sterile distilled water, and then 10XNEB buffer (NEB Inc.), 100XBSA (bovine serum albumin available from NEB Inc.) and 2 µl of SfiI (restriction endonuclease, 20 unit/µl, NEB Inc.) were added. The mixture was reacted overnight at 50 °C to effect SfiI restriction endonuclease treatment. This was followed by phenol/chloroform treatment and ethanol precipitation to obtain a pool of cDNA which had been SfiI-treated at both ends.

### 11. Size fractionation of SfiI-treated cDNA

The SfiI-treated cDNA pool prepared in 10. above was electrophoresed on 1% agarose gel and a fraction with >2 kb was purified using Geneclean II (Bio101 Inc.). The purified cDNA pool was dissolved in 100 µl of sterile distilled water and allowed to stand at 37 °C for 6 hours. This was followed by phenol/chloroform treatment and ethanol precipitation to obtain a long-chain cDNA pool.

### 12. cDNA library

The long-chain cDNA pool prepared in 11. above was ligated into the cloning vector pME18S-FL3 (provided by Prof. Sumio Kanno of the Institute of Medical Science, Tokyo University) using a DNA Ligation Kit ver.1 (kit by Takara Shuzo Co. Ltd.). The long-chain cDNA pool was dissolved in 8 µl of sterile distilled water, and then 1 µl of pME18S-FL3 pretreated with restriction endonuclease DraIII, 80 µl of Solution A (kit accessory) and 10 µl of Solution B (kit accessory) were added and reaction was conducted at 16 °C for 3 hours. This was followed by phenol/chloroform treatment and ethanol precipitation for purification to obtain a cDNA library.

### (Example 2) Transformation into E. coli

### 1. Cloning

The cDNA library prepared in Example 1-12. above was used for transformation into *E. coli* (TOP-10: Invitrogen Corporation). The cDNA library was dissolved in 10µl of sterile distilled water and mixed with TOP-10. The mixture was then incubated on ice for 30 minutes, at 40 °C for 1 minute and on ice for 5 minutes. After adding 500 µl of SOB medium, shake culturing was performed at 37 °C for 60 minutes. Appropriate amounts thereof were seeded onto ampicillin-containing agar media and culturing was continued at 37 °C for a day and a night to obtain *E. coli* clones.

### 2. Preservation of E. coli clones (Preparation of glycerol stock)

The *E. coli* clones on agar media obtained in 1. above were collected with toothpick and suspended in 120 µl of LB medium prepared in a 96-well plate. The 96-well plate was then allowed to stand overnight at 37 °C for culturing of the *E. coli*. A 72 µl portion of 60% glycerol solution was then added and preserved at - 20°C (glycerol stock).

### (Example 2) Sequencing

### 1. Preparation of plasmid

The 10 µl of glycerol stock prepared in Example 1-2 above was transferred to a 15 ml centrifugation tube, and then 3 ml of LB medium and 50 µg/ml of ampicillin were added and shaking was carried out overnight at 37 °C for culturing of the *E. coli.* A QIAprep Spin Miniprep Kit (QIAGEN Inc.) was then used to extract and purify a plasmid DNA from the *E. coli.*

### 2. Analysis of both end sequences

Both end sequences of the plasmid DNA prepared in 1. above were determined using a DNA Sequencing Kit (kit by ABI). There were combined 600 ng of plasmid DNA, 8 µl of premix (kit accessory) and 3.2 pmol of primers, and sterile distilled water was added to a total of 20 µl. After denaturing the mixture at 96 °C for 2 minutes, a cycle of 96 °C for 10 seconds, 50 °C for 5 seconds and 60 °C for 4 minutes was repeated 25 times for reaction. The product was then purified by ethanol precipitation. Sequence determination was carried out by polyaqcrylamide gel electrophoresis under denaturing conditions, using ABI377 (ABI).

### (Example 3) Homology search of database

An internet-mediated base sequence homology search was conducted for the base sequence data obtained from the both end-sequence analysis in Example 2. The search was conducted using the BLAST database of the NCBI (National Center of Biotechnology Information, http://www.ncbi.nblm.nih.gov/BLAST). As a result of the homology search, nbla0078 (one of the cDNA samples) showed high homology to the genomic sequence on human chromosome No. 9 (GeneBank Accession No. AL161625).

### (Example 4) Cloning of the full-length nbla0078

For the genomic sequence obtained in Example 3, its gene transcription sequence was deduced using GENESCAN (Burge C et al.: 1997, 1998) and FGENESH (Salamov AA et al.: 1999). Based on the putative sequence the cloning of the full-length of nbla0078 was conducted according to the method described below.

Specifically, 15 µg of total RNA extracted from a clinical tissue of neuroblastoma with favorable prognosis was reverse transcribed to cDNA using superscript II reverse transcriptase (GIBCO). The reverse-transcribed cDNA (2µl), 5 µl of sterile distilled water, 1 µl of 10XrTaq buffer (Takara Shuzo Co., Ltd.), 1 µl of 2 mM dNTPs, 0.5 µl each of the synthesized primer set and 0.5 µl of rTaq (Takara Shuzo Co., Ltd.) were combined. After denaturing the mixture at 95 °C for 2 minutes, a cycle of 95 °C for 15 seconds, 58 °C for 15 seconds and 72 °C for 20 seconds was repeated 35 times, and then the mixture was allowed to stand at 72 °C for 20 minutes for PCR reaction. The bands amplified by PCR were subcloned into a pGEM-T easy vector (Promega Corporation) and the base sequences were determined according to a standard method (Sanger F. et al.: Proc. Natl. Acad Sci. USA 74: 5463-5467 (1977)). AB1377 (ABI) was used for analysis and both strands of all the base sequence were analyzed.

The gene sequence of NEDL-1 obtained was registered with DDBJ, GeneBank, EMBL. The accession number was AB048365.

### (Example 5) Comparison of gene expression levels in human neuroblastomas with favorable prognosis and unfavorable prognosis by semi-quantitative PCR

All semi-quantitative RT-PCR reactions were performed in the manner described below.

### 1. Reverse transcription (RT)

The extracted total RNA (5 µg) was reverse-transcribed into cDNA using a Superscript II reverse transcriptase (GIBCO).

### 2. PCR

PCR was performed with rTaq (Takara Shuzo Co., Ltd.). The reverse-transcribed cDNA (2µl), 5 µl of sterile distilled water, 1 µl of 10XrTaq buffer, 1 µl of 2 mM dNTPs, 0.5 µl each of the synthesized primer set and 0.5 µl of rTaq were combined. After denaturing the mixture at 95 °C for 2 minutes, a cycle of 95 °C for 15 seconds, 58 °C for 15 seconds and 72 °C for 20 seconds was repeated 35 times, and then the mixture was allowed to stand at 72 °C for 20 minutes for PCR reaction.

GAPDH was used as the positive control. Primers are shown below.

FW: 5'CTGCACCAACAATATCCC3' (SEQ ID NO:3)

RV: 5'GTAGAGACAGGGTTTCAC3' (SEQ ID NO:4)

### 3. Comparison of NEDL-1 gene expression levels

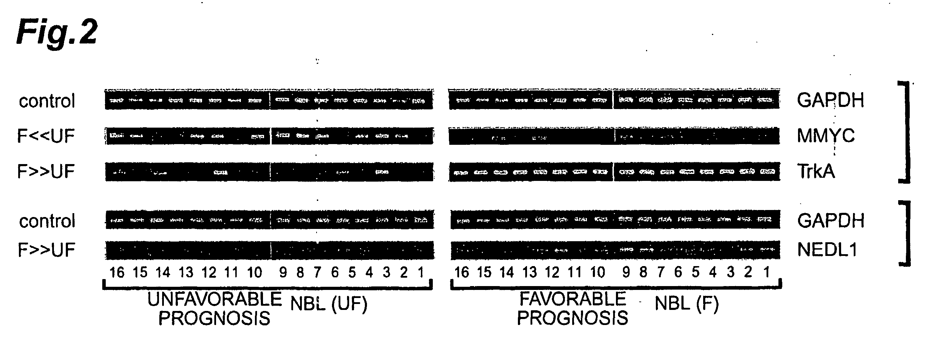

RT-PCR was performed on the total RNAs of neuroblastomas with favorable prognosis and with unfavorable prognosis obtained in Preparation Example 1-3 under the conditions described above. These reaction solutions were electrophoresed on 2.5% agarose gel. The results confirmed that the expression of the NEDL-1 gene was specific for the neuroblastoma clinical tissues with favorable prognosis. Results are shown in Fig. 2. Here, in Fig. 2 the samples in each lane are as follows:
Lanes F1-16 (left): neuroblastoma clinical samples with favorable prognosis
Lanes UF1-16 (right): neuroblastoma clinical samples with unfavorable prognosis
Control: GAPDH
Positive control (favorable prognosis): TrkA
Negative control (unfavorable prognosis): NMYC

### (Example 6) Tissue-dependent gene expression levels by semi-quantitative PCR

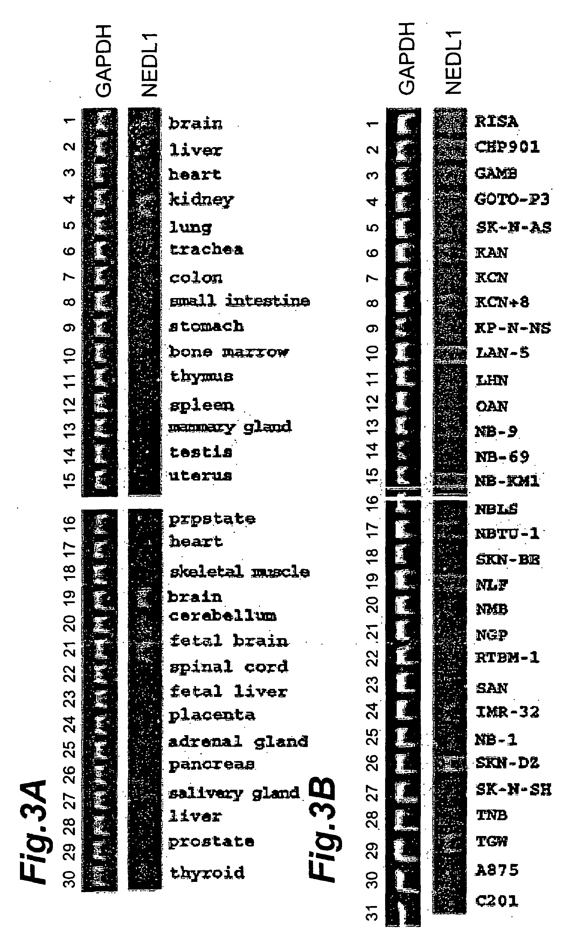

mRNAs of normal human tissues (Clontech) were used to perform RT-PCR under the conditions described in Example 5. These reaction solutions were electrophoresed on 2.5% agarose gel. The results confirmed that the expression of the NEDL-1 gene expression was tissue-specific among the normal human tissues. Results are shown in Fig. 3. The expression of NEDL-1 was restricted in the brain, the fetal brain, the cerebellum and the kidney.

### (Example 7) Gene expression levels that are dependent on neuroblastoma cell lines by semi-quantitative PCR

RT-PCR was performed on the total RNAs of various neuroblastoma cell lines under the conditions described in Example 5. These reaction solutions were electrophoresed on 2.5% agarose gel. The results confirmed that the distribution of NEDL-1 gene expression was tissue-specific. Results are shown in Fig. 3B. Those with which NEDL-1 expression was observed were SKN-DZ, TGW, KAN, KCN+8, and LAN-5.

### (Example 8) Northern hybridization

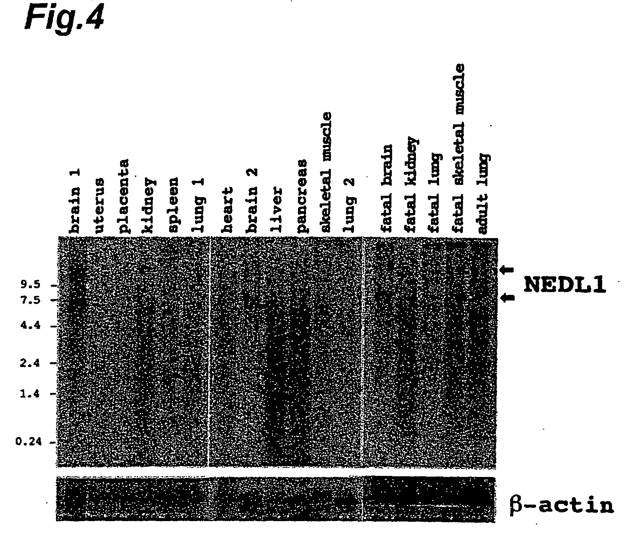

A multi tissue Northern blot on which poly(A)⁺RNA of different human tissues had been blotted was used together with NEDL-1 cDNA (labeled with ³²P) as a probe to carry out hybridization. A β-actin cDNA probe was used as control. Results are shown in Fig. 4. Two transcripts with about 10.0 kb and about 7.0 kb were observed in the brain, the kidney and the fetal brain.

### (Example 9) Ubiquitin ligase activity

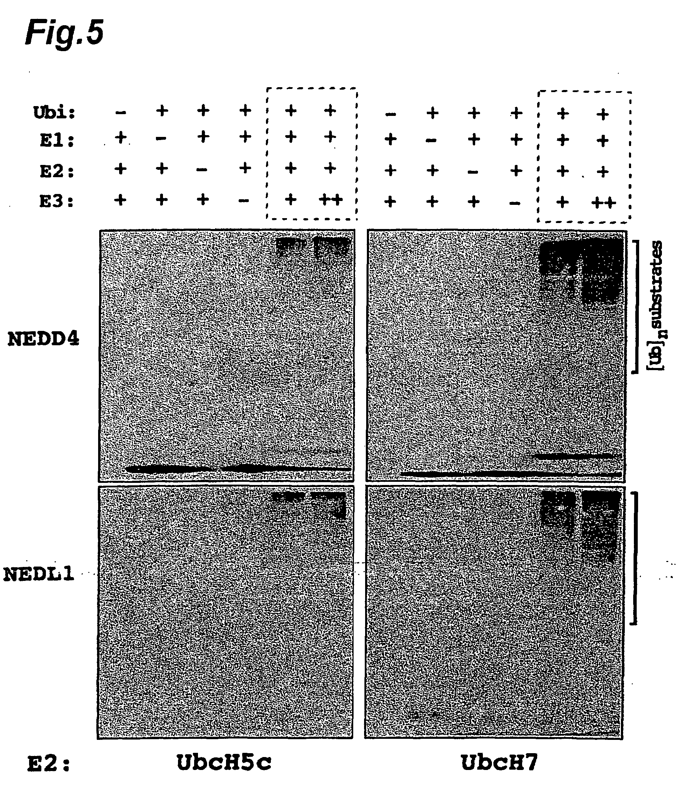

Equivalent amounts of a bacterial lysis product expressing E2 (UbcH5c or UbcH7) were incubated with ubiquitin, yeast E1 and E3 (Nedd4, NEDL-1 or NEDL-2) at 37 °C for 2 hours. Subsequently, the product was separated on SDS-PAGE under reductive conditions and blotted with anti-ubiquitin antibody. Purified recombinant GST-Nedd1, GST-NEDL-1/HECT and GST-NEDL2/HECT were respectively used as E3 (ubiquitin ligase). Results are shown in Fig. 5. Ubiquitination increased depending on the amount of E3 (regions enclosed by dotted line in the figure). NEDL-1 displayed ubiquitin ligase activity at the same level as Nedd4 which served as positive control.

### (Example 10) Cellular localization of NEDL-1

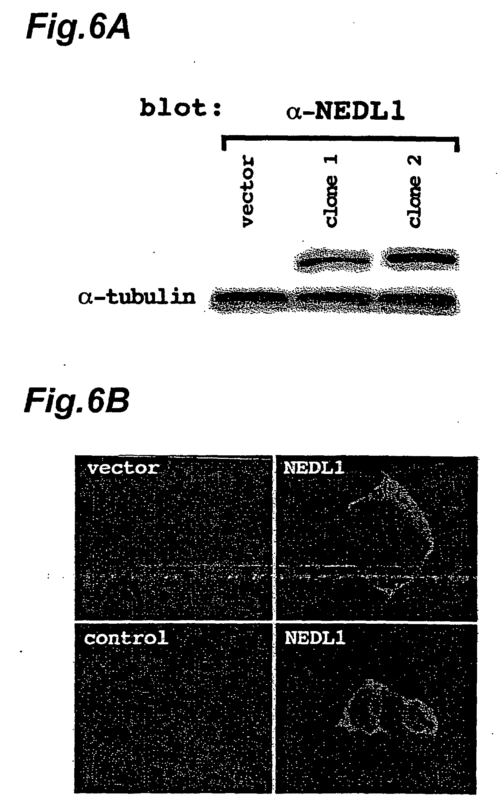

The full-length NEDL-1 gene was transfected into Cos 7 cells transiently. Forty eight hours later, the cells were lysed, subjected to SDS-PAGE on 6% polyaqcrylamide and analyzed with NEDL-1 antibody. Each gene product was detected at the position of about 220 kD. Results are shown in Fig. 6A. In endogenous expression (CHP134 cells) and exogenous expression (Cos 7 cells) NEDL-1 was mainly localized in the cytoplasm and the cell membrane. The result is well in accord with those from the other members of the Nedd4 family (Fig. 6B).

### (Example 11) Interaction between NED-1 and AICD

A typical yeast two-hybrid screening was performed using a MATCHMAKER GAL4 Two-HYBRID SYSTEM2 (K1604-1: Clontech Company) with the NEDL-1 WW domain region as a DNA binding domain fusion protein. Specifically, PCR cloning was carried out in pAS2-1 (GenBank Accession No. U3-4907) in frame and sequencing was carried out with a DNA sequencer ABI PRISM 377 (Perkin Elmer/Applied Biosystems). CG-1945 cell line was used as a directing yeast cell line and a Human fetal Brain MATCHMAKER cDNA Library (Priming Method: Xho I-(dT)15/Vector: pACT2/Cat.#HL4028AH) was used as a library. Proliferation potency was assayed in a SD(-His, -Trp)TPD plate and library screening was performed in a YPD medium with the addition of 3-amino-1,2,4-triazole (20 mM) which was an inhibitor of HIS3. HIS+ colonies were picked up and assayed for their β-galactosidase activity according to a standard method, where positive clones were selected. Plasmid DNAs were collected from these positive clones according to a standard method. Following the above procedure, a clone having the AICD region was found in a plurality of positive clones that had been discovered by performing the yeast two-hybrid screening. The clone was focused and investigation proceeded.

A FLAG sequence (DYKDDDDK) was appended to the ACID region of the clone and a mammalian cell expression vector capable of expression under a CMV promoter was constructed. After sequence confirmation with the sequencer described above, the vector was transiently coexpressed with a CMV-NEDL-1 expression vector in the cells. Thus investigation was carried out to see if the physical interaction could be reproduced in a cell.

Cos7 cells were maintained at 80% confluency in a Dulbecco's modified Eagle's medium containing 10% FBS. Each 6 µg of DNA was used to carry out transient gene transfer according to the Lipofection method. LipofectAMINE plus (Life technologies, Inc.) was used as a liposome agent. Forty eight hours later cells were washed twice with PBS on ice and 1 ml of TNEBuffer (10 mM Tris-HCl, pH 7.8/1% NP40/0.15 M NaCl/l mM EDTA/10 µl aprotinin) was added thereto. Incubation was carried out on ice for 10 minutes. The cells were then transferred to an Eppendorf tube and after addition of 20 µl of Protein B-Sepharose (50% slurry), the cells were revolved at 4 °C for 30 minutes to eliminate non-specific bonding. Then cells were centrifuged at 15,000 rpm/30 minutes at 4 °C. The supernatant was transferred to a new Eppendorf tube by decantation and after addition of 30 µl of Protein B-Sepharose and 10µl of anti-NED 1 antibody, the cells were revolved at 4 °C for 3 hours. The cells were then spanned down and were washed with THE Buffer four times. Subsequently, 25 µl of TNE buffer and 25 µl of x2sample buffer were added to the cells and the mixture was boiled for 5 minutes to prepare samples for electrophoresis. The same amounts of protein were developed on Tricine SDS-PAGE using 15% aqcrylamide gel, transcribed onto a PVDF membrane and blocked with 3% BSA. After antibody reaction with anti-FLAG-M2 antibody (Sigma) as a primary antibody and with antimouse IgG antibody labeled with HRP as a secondary antibody, the proteins were detected with an ECL Western Blotting Detection Reagent (code no. RPN2106).

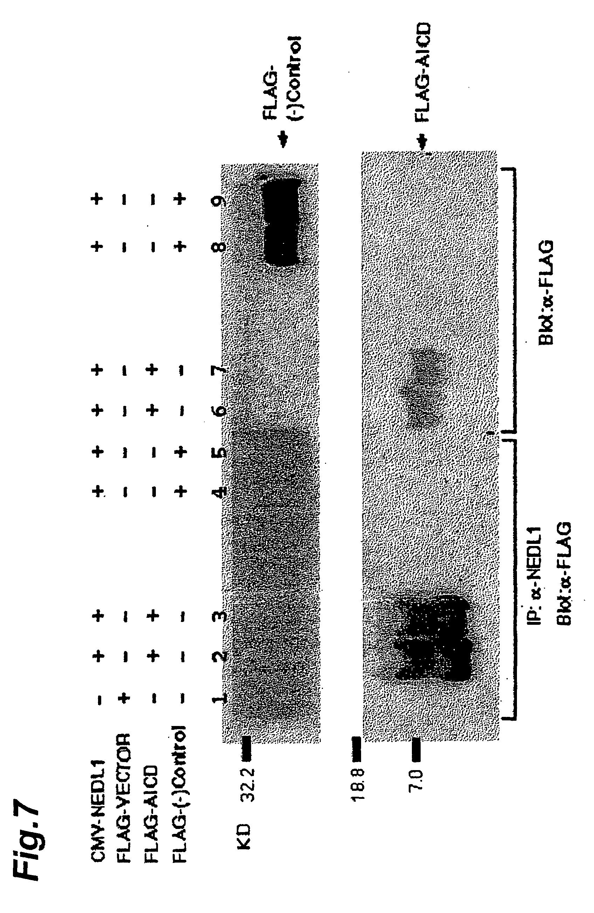

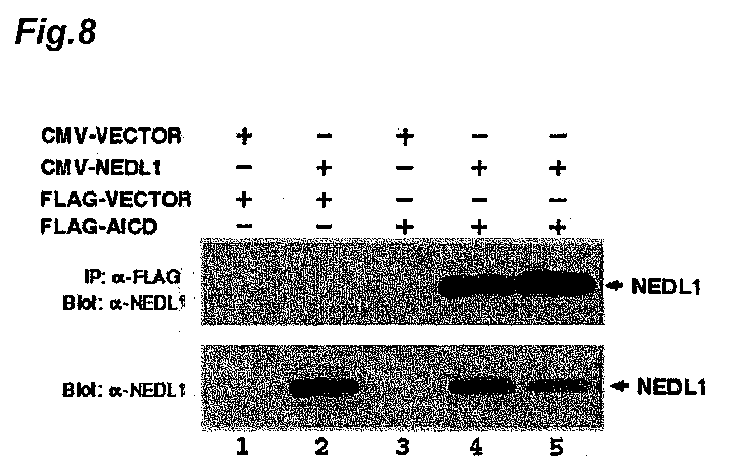

Fig. 7 shows the immunoprecipitation with anti-NEDL-1 antibody followed by the detection with anti-FLAG antibody. Lanes 2 and 3 and Lanes 6 and 7 are a set; Lanes 4 and 5 and Lanes 8 and 9 are a set. The respective sets resulted from two different, independent clones. FLAG-ACID coprecipitated with NEDL-1 (Lanes 2 and 3). The FLAG-AICD protein band expressed is weak in a normal Western Blotting and this is due to the instability of the protein (Lanes 4 and 5). Lanes 4 and 5/8 and 9 are negative controls. According to the normal Western Blotting strong expression was observed in Lanes 8 and 9, while coprecipitation with NEDL-1 was not observed in Lanes 4 and 5. Fig. 8 shows the immunoprecipitation with anti-FLAG antibody followed by the detection with anti-NEDL-antibody. Only where NEDL-1 and FLAG-ACID were coexisted, strong coprecipitation band was obtained (Lanes 4 and 5), which shows direct interaction of the two.

### (Example 12) Ubiquitination of BAPP and ACID by NEDL-1

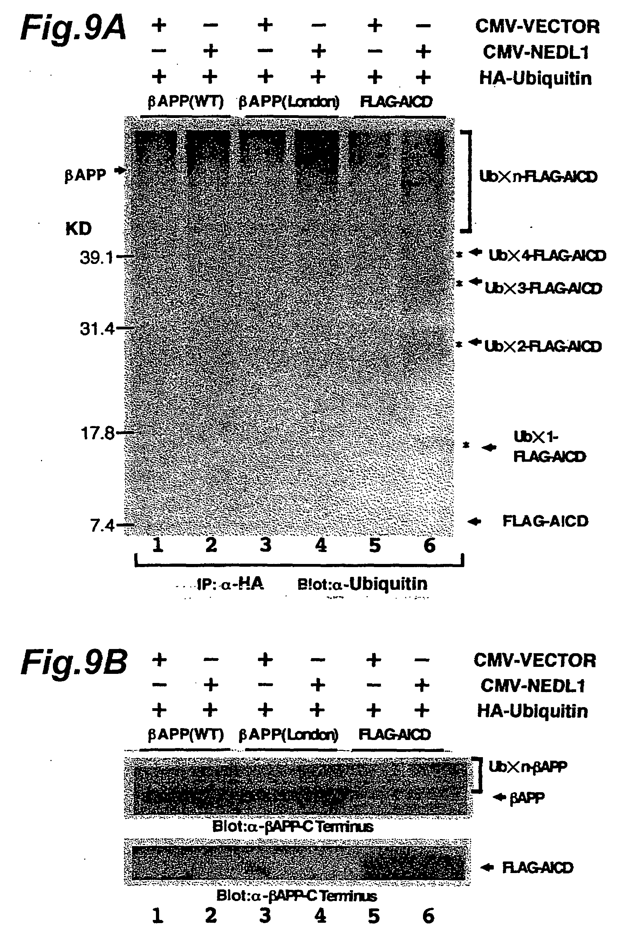

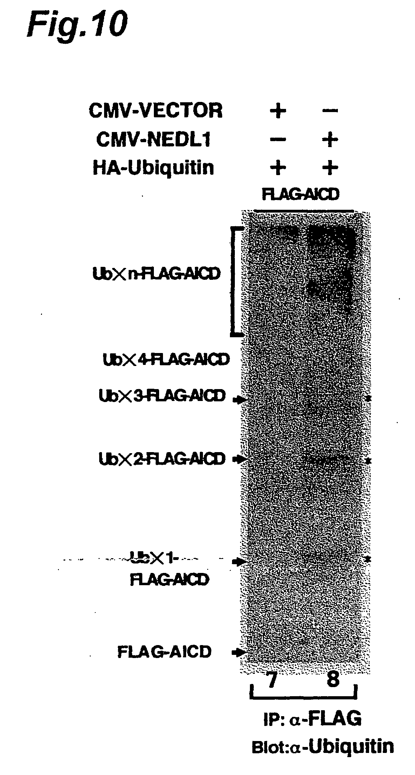

Yeast two-hybrid screening as described in Example 1 was performed, except that prior to harvesting treatment with MG132 (20 µM), a proteasome inhibitor, was done for 2 hours. The other experimental procedures followed almost Example 11. Figs. 9 and 10 show the results of immunoprecipitation.

Fig. 9A shows the immunoprecipitation with anti-HA antibody followed by blotting with anti-ubiquitin antibody. It can be seen that the absolute amount of the ubiquitinated molecules in the cell has increased in the presence of NEDL-1. Fig. 10 shows the immunoprecipitation with anti-FLAG antibody followed by blotting with anti-ubiquitin antibody. Fig. 9B shows the immunoprecipitation with anti-HA antibody followed by blotting with an antibody recognizing ACID. A high molecular weight smear band was observed upward starting from βAPP. This suggests the ubiquitination of βAPP. From Figs. 9A and 9B it is obvious that β APP has been subjected to ubiquitination in the presence of NEDL-1. However, its degree is not related to the mutation of βAPP (whether WT or MT) (Lanes 1 and 2, and Lanes 3 and 4 both in Fig. 9A and Fig. 9B). Lanes 5 and 6 in Fig. 9A and Lanes 7 and 8 in Fig. 10 show the ubiquitination of AICD. FLG-ACID has a molecular weight of about 7 KD (Fig. 9B). Anti-ubiquitin antibody was used for detection in Fig. 9A and Fig. 10, where FLG-ACID that was not ubiquitinated does not appear (lowest column). As a ubiquitin molecule with about 9 kD adds to FLAG-AICD, bands increasing by the about 9 kD are detected with anti-ubiquitin antibody (indicated as asterisks in the figure). It is understood that ACID alone is subjected to ubiquitination by NEDL-1. In the figure, bands that look two-lines represent the differential molecular weight between the exogenous ubiquitin with a HA tag added and the endogenous ubiquitin with no tag.

### (Example 13) Interaction between NEDL-1 and SOD1 mutants

The yeast two-hybrid screening as described in Example 11 was performed on SOD1 genes. The experimental procedure generally followed Example 11. Specifically, SOD1 genes (wild type and mutant types) were transiently coexpressed with CMV-NEDL-1 in cells.

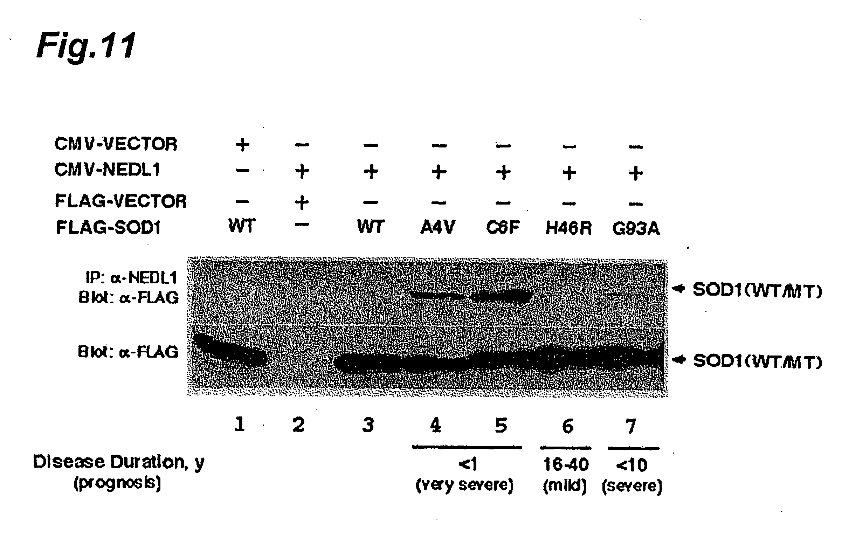

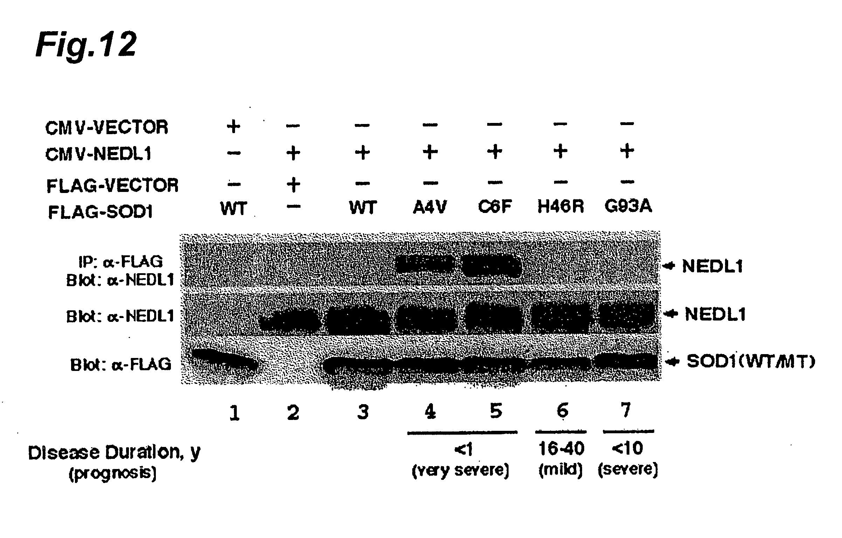

After immunoprecipitation with anti-NEDL-1 antibody, the cells were washed to prepare samples for electrophoresis. The same amounts of protein were developed on 15% SDS-PAGE, transcribed onto a PVDF membrane and allowed for antibody reaction with anti-FLAG antibody, after which detection by ECL was carried out. Results are shown in Fig. 11. Similarly, after immunoprecipitation with anti-FLAG antibody, the cells were washed to prepare samples for electrophoresis. The same amounts of protein were developed on 6% SDS-PAGE, transcribed onto a PVDF membrane and allowed for antibody reaction with anti-NEDL-1 antibody, after which detection by ECL was carried out. Results are shown in Fig. 12. In Lane 3 NEDL-1 and SOD1(WT) did not coprecipitate. In Lanes 4 and 5 SOD1(A4V) and SOD1(C6F) strongly interacted with NEDL-1 and coprecipitated: SOD1(A4V) and SOD1(C6F) were mutants that caused rapid clinical progress after crisis and death within one year. In Lane 6 SOD1(H46R) showed very week interaction with NEDL-1: SOD1(H46R) displayed slow clinical progress after crisis and made survival of nearly 40 years possible. In Lane 7 SOD1(G93A) showed medium interaction with NEDL-1: SOD1(G93A) was a mutant that displayed s peculiar neural symptom after crisis.

The test results shown in Figs. 11 and 12 have revealed that NEDL-1 does not interact with SOD1 of the wild type but interacts with the SOD1 mutants which are responsible for familial ALS. Further, it has been found that the degree of interaction roughly correlates with the degree of clinical malignancy.

### (Example 14) Ubiquitination of SOD1 mutants by NEDL-1

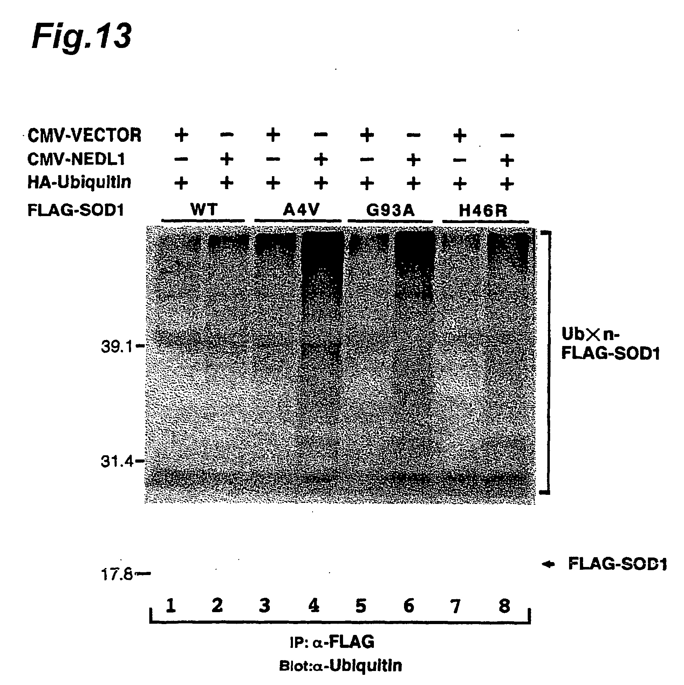

The yeast two-hybrid screening as described in Example 1 was performed, except that prior to harvesting treatment with MG132 (20 µM), a proteasome inhibitor, was done for 2 hours. The other experimental procedures almost followed Example 11. Figs. 13 show the results of immunoprecipitation, where the products were immunoprecipitated with FLAG and then blotted with anti-ubiquitin antibody. The SOD1 mutants were ubiquitinated in the absence of NEDL-1. See Lanes 1, 3, 5 and 7 in Fig. 13. The degree of ubiquitination is 3>5>7>1; it correlates with the clinical severity of the mutants (as explained above). This suggests the possibility that a ubiquitin ligase (such as dorfin) existing in a cell to manage quality control is involved. (Niwa J., Ishigaki S., Doyu M., Suzuki T., Tanaka K., Sobue G. A., Novel centrosomal ring-finger protein, dorfin, mediates ubiquitin ligase activity. Biochem. Biophys. Res Commun. 2001 Mar 2; 281 (3): 706-13.)

It is also understood that the degree of ubiquitination drastically increases in the presence of NEDL-1. See Lanes 2, 4, 6, and 8 in Fig. 13. The ubiquitination degree is again 4>6>8>2 and correlates with the clinical severity of the mutants. It is thus understood that NEDL-1 has the function, as ubiquitin ligase of the quality control type, of strongly exhibiting the ubiquitination power against SOD1 mutants as opposed to mutant BAPP.

### Industrial Applicability

As described above, the nucleic acid probes and primers according to this invention may be used for various types of hybridization or PCR, and permit detection of the expression of the NEDL-1 gene in not only neuroblastomas but also other human tissues and cells, as well as the analysis of its structure and function. Production of the NEDL-1 protein encoded by the gene through genetic engineering is also possible according to the invention. The protein has been confirmed for its ubiquitin ligase activity and has been shown to be a ubiquitin ligase of the HECT type based on its structure. Accordingly, the identification of the substrate for the NEDL-1 protein in the ubiquitin-proteasome system has been possible and it will lead to the possibility of treating neurodegenerative diseases involving the protein. In reality, it was determined that NEDL-1 interacted with β APP, AICD or SOD1 (mutant type). Further, this interaction was identified to be through ubiquitination.

The nucleic acids according to this invention are those derived from the NEDL-1 gene whose expression is enhanced in neuroblastoma with favorable prognosis, and therefore allow the diagnosis for the prognosis of neuroblastoma based on this genetic information from these nucleic acids. Unlike the N-myc gene which is a factor for unfavorable prognosis, these genes are considered factors for favorable prognosis, similarly to the TrkA gene, and therefore can serve as markers (tumor markers) for neuroblastoma malignancy and sensitivity to anti-cancer agents. Specifically, the nucleic acid probes of this invention or the primers of the invention may be used to construct the diagnostic agents or diagnostic kits for the prognosis of neuroblastoma and to detect the NEDL-1 protein or the NEDL-1 protein in clinical tissue samples, whereby the prognosis can be diagnosed.

## Claims

1. A nucleic acid probe comprising nucleic acid
(a) or nucleic acid (b):
(a) a nucleic acid having a portion of a base sequence set forth in SEQ ID NO:2 in the Sequence Listing or a base sequence complementary thereto; and
(b) a nucleic acid capable of hybridizing to the nucleic acid comprising a base sequence set forth in SEQ ID NO:2 in the Sequence Listing, or having a base sequence complementary to said base sequence.

2. The nucleic acid probe according to claim 1, wherein the nucleic acid is DNA.

3. The nucleic acid probe according to claim 1 or 2, wherein the nucleic acid has a base length of at least 20 bases.

4. The nucleic acid probe according to claim 3, wherein the base sequence set forth in SEQ ID NO:2 is a full-length thereof.

5. A diagnostic agent for the prognosis of neuroblastoma, the agent comprising the nucleic acid probe according to any of claims 1-4, as the effective ingredient.

6. A primer containing DNA (a) or DNA (b):
(a) DNA having a portion of a base sequence set forth in SEQ ID NO:2 in the Sequence Listing or a base sequence complementary thereto; or
(b) DNA capable of hybridizing to the DNA comprising a base sequence set forth in SEQ ID NO:2 in the Sequence Listing, or having a base sequence complementary to said base sequence.

7. A kit for diagnosing the prognosis of neuroblastoma, the kit comprising the primer according to claim 6 as the effective ingredient.

8. A method for diagnosing the prognosis of neuroblastoma, the method comprising detecting the presence or absence of a nucleic acid comprising a base sequence set forth in SEQ ID NO:2 in the Sequence Listing in a clinical tissue sample of neuroblastoma.

9. A method for diagnosing the prognosis of neuroblastoma, the method comprising detecting the presence or absence of a protein comprising an amino acid sequence set forth in SEQ ID NO:1 in the Sequence Listing in a clinical tissue sample of neuroblastoma.

10. A method for diagnosing the prognosis of neuroblastoma, the method comprising contacting with a clinical tissue sample of neuroblastoma, (a) a nucleic acid having a portion of a base sequence set forth in SEQ ID NO:2 in the Sequence Listing or a base sequence complementary thereto or (b) a nucleic acid capable of hybridizing to the nucleic acid comprising a base sequence set forth in SEQ ID NO:2 in the Sequence Listing, or having a base sequence complementary to said base sequence; and analyzing the expression of a protein comprising an amino acid sequence set forth in SEQ ID NO:1 in the Sequence Listing or an level thereof.

11. A polyubiqutination agent comprising as the effective ingredient, a protein comprising a base sequence set forth in SEQ ID NO: 1 in the Sequence Listing.

12. The polyubiqutination agent according to claim 11, wherein the substrate to be ubiquitinated is β-amyloid precursor protein (βAPP).

13. The polyubiqutination agent according to claim 11, wherein the substrate to be ubiquitinated is β-amyloid precursor protein intracellular region (AICD)

14. The polyubiqutination agent according to claim 11, wherein the substrate to be ubiquitinated is a superoxide dismutase mutant (SOD1).

15. A composition for modulating β-amyloid precursor protein (βAPP), the composition comprising an effective amount of a protein comprising an amino acid sequence set forth in SEQ ID NO:1 in the Sequence Listing to modulate the expression, the production or the formation of β-amyloid precursor protein in a cell.

16. A composition for modulating β-amyloid precursor protein (βAPP), the composition comprising an effective amount of a nucleic acid comprising a base sequence set forth in SEQ ID NO:2 in the Sequence Listing to modulate the expression, the production or the formation of β-amyloid precursor protein in a cell.

17. A method for modulating the expression, the production or the formation of β-amyloid precursor protein in a cell, the method comprising administering an effective amount of a protein comprising an amino acid sequence set forth in SEQ ID NO:1 in the Sequence Listing to modulate the expression, the production or the formation of β-amyloid precursor protein in a cell.

18. A method for modulating the expression, the production or the formation of β-amyloid precursor protein in a cell, the method comprising administering an effective amount of a nucleic acid comprising a base sequence set forth in SEQ ID NO:2 in the Sequence Listing to modulate the expression, the production or the formation of β-amyloid precursor protein in a cell.

19. A composition for modulating superoxide dismutase (SOD1) activity, the composition comprising an effective amount of a protein comprising an amino acid sequence set forth in SEQ ID NO:1 in the Sequence Listing to modulate the superoxide dismutase (SOD1) activity in a cell.

20. A composition for modulating superoxide dismutase (SOD1) activity, the composition comprising an effective amount of a nucleic acid comprising a base sequence set forth in SEQ ID NO:1 in the Sequence Listing to modulate the superoxide dismutase (SOD1) activity in a cell.

21. The composition for modulating superoxide dismutase (SOD1) activity according to claim 19 or 20, wherein the superoxide dismutase (SOD1) is a mutant type.

22. A method for modulating superoxide dismutase activity in a cell, the method comprising administering to the cell, an effective amount of a protein comprising an amino acid sequence set forth in SEQ ID NO:1 in the Sequence Listing to modulate the superoxide dismutase (SOD1) activity.

23. A method for modulating superoxide dismutase (SOD1) activity in a cell, the method comprising administering to the cell, an effective amount of a nucleic acid comprising a base sequence set forth in SEQ ID NO:2 in the Sequence Listing to modulate the superoxide dismutase (SOD1) activity.

24. The method according to claim 22 or 23, wherein the superoxide dismutase (SOD1) is a mutant type.


SEQUENCE LISTING

<110> Hisamitsu Pharmaceutical Co., Inc.

<120> Novel gene NEDL-1

<130> FP02-0209-00WO

<140>
<141>

<150> JP 2002-116753
<151> 2002-4-18

<150> JP 2001-254974
<151> 2001-8-24

<160> 4

<170> PatentIn Ver. 2.1

<210> 1
<211> 1585
<212> PRT
<213> Artificial Sequence

<220>

<223> Description of Artificial Sequence:synthetic

      polynucleotide

<400> 1

Met Ala Ser Pro Ser Arg Asn Ser Gln Ser Arg Arg Arg Cys Lys Glu

 1               5                   10                  15

Pro Leu Arg Tyr Ser Tyr Asn Pro Asp Gln Phe His Asn Met Asp Leu

            20                  25                  30

Arg Gly Gly Pro His Asp Gly Val Thr Ile Pro Arg Ser Thr Ser Asp

        35                  40                  45

Thr Asp Leu Val Thr Ser Asp Ser Arg Ser Thr Leu Met Val Ser Ser

    50                  55                  60

Ser Tyr Tyr Ser Ile Gly His Ser Gln Asp Leu Val Ile His Trp Asp

65                  70                  75                  80

Ile Lys Glu Glu Val Asp Ala Gly Asp Trp Ile Gly Met Tyr Leu Ile

                85                  90                  95

Asp Glu Val Leu Ser Glu Asn Phe Leu Asp Tyr Lys Asn Arg Gly Val

            100                 105                 110

Asn Gly Ser His Arg Gly Gln Ile Ile Trp Lys Ile Asp Ala Ser Ser
        115                 120                 125

Tyr Phe Val Glu Pro Glu Thr Lys Ile Cys Phe Lys Tyr Tyr His Gly
    130                 135                 140

Val Ser Gly Ala Leu Arg Ala Thr Thr Pro Ser Val Thr Val Lys Asn
145                 150                 155                 160

Ser Ala Ala Pro Ile Phe Lys Ser Ile Gly Ala Asp Glu Thr Val Gln
                165                 170                 175

Gly Gln Gly Ser Arg Arg Leu Ile Ser Phe Ser Leu Ser Asp Phe Gln
            180                 185                 190

Ala Met Gly Leu Lys Lys Gly Met Phe Phe Asn Pro Asp Pro Tyr Leu
        195                 200                 205

Lys Ile Ser Ile Gln Pro Gly Lys His Ser Ile Phe Pro Ala Leu Pro
    210                 215                 220

His His Gly Gln Glu Arg Arg Ser Lys Ile Ile Gly Asn Thr Val Asn
225                 230                 235                 240

Pro Ile Trp Gln Ala Glu Gln Phe Ser Phe Val Ser Leu Pro Thr Asp
                245                 250                 255

Val Leu Glu Ile Glu Val Lys Asp Lys Phe Ala Lys Ser Arg Pro Ile
            260                 265                 270
Ile Lys Arg Phe Leu Gly Lys Leu Ser Met Pro Val Gln Arg Leu Leu
        275                 280                 285
Glu Arg His Ala Ile Gly Asp Arg Val Val Ser Tyr Thr Leu Gly Arg
    290                 295                 300
Arg Leu Pro Thr Asp His Val Ser Gly Gln Leu Gln Phe Arg Phe Glu
305                 310                 315                 320
Ile Thr Ser Ser Ile His Pro Asp Asp Glu Glu Ile Ser Leu Ser Thr
                325                 330                 335
Glu Pro Glu Ser Ala Gln Ile Gln Asp Ser Pro Met Asn Asn Leu Met
            340                 345                 350
Glu Ser Gly Ser Gly Glu Pro Arg Ser Glu Ala Pro Glu Ser Ser Glu
        355                 360                 365
Ser Trp Lys Pro Glu Gln Leu Gly Glu Gly Ser Val Pro Asp Arg Pro
    370                 375                 380
Gly Asn Gln Ser Ile Glu Leu Ser Arg Pro Ala Glu Glu Ala Ala Val

385                 390                 395                 400

Ile Thr Glu Ala Gly Asp Gln Gly Met Val Ser Val Gly Pro Glu Gly
                405                 410                 415

Ala Gly Glu Leu Leu Ala Gln Val Gln Lys Asp Ile Gln Pro Ala Pro
            420                 425                 430

Ser Ala Glu Glu Leu Ala Glu Gln Leu Asp Leu Gly Glu Glu Ala Ser
        435                 440                 445

Ala Leu Leu Leu Glu Asp Gly Glu Ala Pro Ala Ser Thr Lys Glu Glu
    450                 455                 460

Pro Leu Glu Glu Glu Ala Thr Thr Gln Ser Arg Ala Gly Arg Glu Glu
465                 470                 475                 480

Glu Glu Lys Glu Gln Glu Glu Glu Gly Asp Val Ser Thr Leu Glu Gln
                485                 490                 495

Gly Glu Gly Arg Leu Gln Leu Arg Ala Ser Val Lys Arg Lys Ser Arg
            500                 505                 510

Pro Cys Ser Leu Pro Val Ser Glu Leu Glu Thr Val Ile Ala Ser Ala
        515                 520                 525

Cys Gly Asp Pro Glu Thr Pro Arg Thr His Tyr Ile Arg Ile His Thr
    530                 535                 540

Leu Leu His Ser Met Pro Ser Ala Gln Gly Gly Ser Ala Ala Glu Glu
545                 550                 555                 560

Glu Asp Gly Ala Glu Glu Glu Ser Thr Leu Lys Asp Ser Ser Glu Lys
                565                 570                 575

Asp Gly Leu Ser Glu Val Asp Thr Val Ala Ala Asp Pro Ser Ala Leu
            580                 585                 590

Glu Glu Asp Arg Glu Glu Pro Glu Gly Ala Thr Pro Gly Thr Ala His
        595                 600                 605

Pro Gly His Ser Gly Gly His Phe Pro Ser Leu Ala Asn Gly Ala Ala
    610                 615                 620

Gln Asp Gly Asp Thr His Pro Ser Thr Gly Ser Glu Ser Asp Ser Ser
625                 630                 635                 640

Pro Arg Gln Gly Gly Asp His Ser Cys Glu Gly Cys Asp Ala Ser Cys
                645                 650                 655

Cys Ser Pro Ser Cys Tyr Ser Ser Ser Cys Tyr Ser Thr Ser Cys Tyr
            660                 665                 670

Ser Ser Ser Cys Tyr Ser Ala Ser Cys Tyr Ser Pro Ser Cys Tyr Asn
        675                 680                 685

Gly Asn Arg Phe Ala Ser His Thr Arg Phe Ser Ser Val Asp Ser Ala
    690                 695                 700

Lys Ile Ser Glu Ser Thr Val Phe Ser Ser Gln Asp Asp Glu Glu Glu
705                 710                 715                 720

Glu Asn Ser Ala Phe Glu Ser Val Pro Asp Ser Met Gln Ser Pro Glu
                725                 730                 735

Leu Asp Pro Glu Ser Thr Asn Gly Ala Gly Pro Trp Gln Asp Glu Leu
            740                 745                 750

Ala Ala Pro Ser Gly His Val Glu Arg Ser Pro Glu Gly Leu Glu Ser
        755                 760                 765

Pro Val Ala Gly Pro Ser Asn Arg Arg Glu Gly Glu Cys Pro Ile Leu
    770                 775                 780

His Asn Ser Gln Pro Val Ser Gln Leu Pro Ser Leu Arg Pro Glu His
785                 790                 795                 800

His His Tyr Pro Thr Ile Asp Glu Pro Leu Pro Pro Asn Trp Glu Ala

                805                 810                 815

Arg Ile Asp Ser His Gly Arg Val Phe Tyr Val Asp His Val Asn Arg

            820                 825                 830

Thr Thr Thr Trp Gln Arg Pro Thr Ala Ala Ala Thr Pro Asp Gly Met

        835                 840                 845

Arg Arg Ser Gly Ser Ile Gln Gln Met Glu Gln Leu Asn Arg Arg Tyr

    850                 855                 860

Gln Asn Ile Gln Arg Thr Ile Ala Thr Glu Arg Ser Glu Glu Asp Ser

865                 870                 875                 880

Gly Ser Gln Ser Cys Glu Gln Ala Pro Ala Gly Gly Gly Gly Gly Gly

                885                 890                 895

Gly Ser Asp Ser Glu Ala Glu Ser Ser Gln Ser Ser Leu Asp Leu Arg

            900                 905                 910

Arg Glu Gly Ser Leu Ser Pro Val Asn Ser Gln Lys Ile Thr Leu Leu

        915                 920                 925

Leu Gln Ser Pro Ala Val Lys Phe Ile Thr Asn Pro Glu Phe Phe Thr

    930                 935                 940

Val Leu His Ala Asn Tyr Ser Ala Tyr Arg Val Phe Thr Ser Ser Thr
945                 950                 955                 960

Cys Leu Lys His Met Ile Leu Lys Val Arg Arg Asp Ala Arg Asn Phe
                965                 970                 975

Glu Arg Tyr Gln His Asn Arg Asp Leu Val Asn Phe Ile Asn Met Phe
            980                 985                 990

Ala Asp Thr Arg Leu Glu Leu Pro Arg Gly Trp Glu Ile Lys Thr Asp
        995                 1000                1005

Gln Gln Gly Lys Ser Phe Phe Val Asp His Asn Ser Arg Ala Thr Thr
    1010                1015                1020

Phe Ile Asp Pro Arg Ile Pro Leu Gln Asn Gly Arg Leu Pro Asn His
1025                1030                1035                1040

Leu Thr His Arg Gln His Leu Gln Arg Leu Arg Ser Tyr Ser Ala Gly
                1045                1050                1055

Glu Ala Ser Glu Val Ser Arg Asn Arg Gly Ala Ser Leu Leu Ala Arg
            1060                1065                1070

Pro Gly His Ser Leu Val Ala Ala Ile Arg Ser Gln His Gln His Glu
        1075                1080                1085

Ser Leu Pro Leu Ala Tyr Asn Asp Lys Ile Val Ala Phe Leu Arg Gln
    1090                1095                1100
Pro Asn Ile Phe Glu Met Leu Gln Glu Arg Gln Pro Ser Leu Ala Arg
1105                1110                1115                1120
Asn His Thr Leu Arg Glu Lys Ile His Tyr Ile Arg Thr Glu Gly Asn
                1125                1130                1135
His Gly Leu Glu Lys Leu Ser Cys Asp Ala Asp Leu Val Ile Leu Leu
            1140                1145                1150
Ser Leu Phe Glu Glu Glu Ile Met Ser Tyr Val Pro Leu Gln Ala Ala
        1155                1160                1165
Phe His Pro Gly Tyr Ser Phe Ser Pro Arg Cys Ser Pro Cys Ser Ser
    1170                1175                1180
Pro Gln Asn Ser Pro Gly Leu Gln Arg Ala Ser Ala Arg Ala Pro Ser
1185                1190                1195                1200
Pro Tyr Arg Arg Asp Phe Glu Ala Lys Leu Arg Asn Phe Tyr Arg Lys
                1205                1210                1215
Leu Glu Ala Lys Gly Phe Gly Gln Gly Pro Gly Lys Ile Lys Leu Ile

            1220                1225                1230

Ile Arg Arg Asp His Leu Leu Glu Gly Thr Phe Asn Gln Val Met Ala
        1235                1240                1245

Tyr Ser Arg Lys Glu Leu Gln Arg Asn Lys Leu Tyr Val Thr Phe Val
    1250                1255                1260

Gly Glu Glu Gly Leu Asp Tyr Ser Gly Pro Ser Arg Glu Phe Phe Phe
1265                1270                1275                1280

Leu Leu Ser Gln Glu Leu Phe Asn Pro Tyr Tyr Gly Leu Phe Glu Tyr
                1285                1290                1295

Ser Ala Asn Asp Thr Tyr Thr Val Gln Ile Ser Pro Met Ser Ala Phe
            1300                1305                1310

Val Glu Asn His Leu Glu Trp Phe Arg Phe Ser Gly Arg Ile Leu Gly
        1315                1320                1325

Leu Ala Leu Ile His Gln Tyr Leu Leu Asp Ala Phe Phe Thr Arg Pro
    1330                1335                1340

Phe Tyr Lys Ala Leu Leu Arg Leu Pro Cys Asp Leu Ser Asp Leu Glu
1345                1350                1355                1360

Tyr Leu Asp Glu Glu Phe His Gln Ser Leu Gln Trp Met Lys Asp Asn
                1365                1370                1375
Asn Ile Thr Asp Ile Leu Asp Leu Thr Phe Thr Val Asn Glu Glu Val
            1380                1385                1390
Phe Gly Gln Val Thr Glu Arg Glu Leu Lys Ser Gly Gly Ala Asn Thr
        1395                1400                1405
Gln Val Thr Glu Lys Asn Lys Lys Glu Tyr Ile Glu Arg Met Val Lys
    1410                1415                1420
Trp Arg Val Glu Arg Gly Val Val Gln Gln Thr Glu Ala Leu Val Arg
1425                1430                1435                1440
Gly Phe Tyr Glu Val Val Asp Ser Arg Leu Val Ser Val Phe Asp Ala
                1445                1450                1455
Arg Glu Leu Glu Leu Val Ile Ala Gly Thr Ala Glu Ile Asp Leu Asn
            1460                1465                1470
Asp Trp Arg Asn Asn Thr Glu Tyr Arg Gly Gly Tyr His Asp Gly His
        1475                1480                1485
Leu Val Ile Arg Trp Phe Trp Ala Ala Val Glu Arg Phe Asn Asn Glu
    1490                1495                1500

Gln Arg Leu Arg Leu Leu Gln Phe Val Thr Gly Thr Ser Ser Val Pro
1505                1510                1515                1520

Tyr Glu Gly Phe Ala Ala Leu Arg Gly Ser Asn Gly Leu Arg Arg Phe
                1525                1530                1535

Cys Ile Glu Lys Trp Gly Lys Ile Thr Ser Leu Pro Arg Ala His Thr
            1540                1545                1550

Cys Phe Asn Arg Leu Asp Leu Pro Pro Tyr Pro Ser Tyr Ser Met Leu
        1555                1560                1565

Tyr Glu Lys Leu Leu Thr Ala Val Glu Glu Thr Ser Thr Phe Gly Leu
    1570                1575                1580

Glu
1585

<210> 2
<211> 6200
<212> DNA
<213> Artificial Sequence

<220>

<223> Description of Artificial Sequence:synthetic
      polynucleotide

<400> 2
ggtttttagg cctggccgcc atggcgtctc cttctagaaa ctcccagagc cgacgccggt 60
gcaaggagcc gctccgatac agctacaacc ccgaccagtt ccacaacatg gacctcaggg 120
gcggccccca cgatggcgtc accattcccc gctccaccag cgacactgac ctggtcacct 180
cggacagccg ctccacgctc atggtcagca gctcctacta ttccatcggg cactctcagg 240
acctggtcat ccactgggac ataaaggagg aagtggacgc tggggactgg attggcatgt 300
acctcattga tgaggtcttg tccgaaaact ttctggacta taaaaaccgt ggagtcaatg 360
gttctcatcg gggccagatc atctggaaga tcgatgccag ctcgtacttt gtggaacctg 420
aaactaagat ctgcttcaaa tactaccatg gagtgagtgg ggccctgcga gcaaccaccc 480
ccagtgtcac ggtcaaaaac tcggcagctc ctatttttaa aagcattggt gctgatgaga 540
ccgtccaagg acaaggaagt cggaggctga tcagcttctc tctctcagat ttccaagcca 600
tggggttgaa gaaagggatg tttttcaacc cagaccctta tctgaagatt tccattcagc 660
ctgggaaaca cagcatcttc cccgccctcc ctcaccatgg acaggagagg agatccaaga 720
tcataggcaa caccgtgaac cccatctggc aggccgagca attcagtttt gtgtccttgc 780
ccactgacgt gctggaaatt gaggtgaagg acaagtttgc caagagccgc cccatcatca 840
agcgcttctt gggaaagctg tcgatgcccg ttcaaagact cctggagaga cacgccatag 900
gggatagggt ggtcagctac acacttggcc gcaggcttcc aacagatcat gtgagtggac 960
agctgcaatt ccgatttgag atcacttcct ccatccaccc agatgatgag gagatttccc 1020
tgagtaccga gcctgagtca gcccaaattc aggacagccc catgaacaac ctgatggaaa 1080
gcggcagtgg ggaacctcgg tctgaggcac cagagtcctc tgagagctgg aagccagagc 1140
agctgggtga gggcagtgtc cccgatcgtc cagggaacca aagcatagag ctttccagac 1200
cagctgagga agcagcagtc atcacggagg caggagacca gggcatggtc tctgtgggac 1260
ctgaaggggc tggggagctc ctggcccagg tgcaaaagga catccagcct gcccccagtg 1320

cagaagagct ggccgagcag ctggacctgg gtgaggaggc atcagcactg ctgctggaag 1380

acggtgaagc cccagccagc accaaggagg agcccttgga ggaggaagca acgacccaga 1440

gccgggctgg aagggaagaa gaggagaagg agcaggagga ggagggagat gtgtccaccc 1500

tggagcaggg agagggcagg ctgcagctgc gggcctcggt gaagagaaaa agcaggccct 1560

gctccttgcc tgtgtccgag ctggagacgg tgatcgcgtc agcctgcggg gaccccgaga 1620

ccccgcggac acactacatc cgcatccaca ccctgctgca cagcatgccc tccgcccagg 1680

gcggcagcgc ggcagaggag gaggacggcg cggaggagga gtccaccctc aaggactcct 1740

cggagaagga tgggctcagc gaggtggaca cggtggccgc tgacccgtct gccctggaag 1800

aggacagaga agagcccgag ggggctactc caggcacggc gcaccctggc cactccgggg 1860

gccacttccc cagcctggcc aatggcgcgg cccaggatgg cgacacgcac cccagcaccg 1920

ggagcgagag cgactccagc cccaggcaag gcggggacca cagttgcgag ggctgtgacg 1980

cgtcctgctg cagcccctcg tgctacagct cctcgtgcta cagcacgtcc tgctacagca 2040

gctcgtgcta cagcgcctcg tgctacagcc cctcctgcta caacggcaac aggttcgcca 2100

gccacacgcg cttctcctcc gtggacagcg ccaagatctc cgagagcacg gtcttctcct 2160

cgcaagacga cgaggaggag gagaacagcg cgttcgagtc ggtacccgac tccatgcaga 2220

gccctgagct ggacccggag tccacgaacg gcgctgggcc gtggcaagac gagctggccg 2280

cccctagcgg gcacgtggaa agaagcccgg aaggtctgga atcccccgtg gcaggtccaa 2340

gcaatcggag agaaggtgaa tgtcctatac tccataattc ccagccagta agccagcttc 2400

cttccctgag gcctgaacat catcactacc caacaatcga tgagcctctt ccaccaaact 2460

gggaagctcg aattgacagc cacgggcggg tcttttatgt ggaccacgtg aaccgcacaa 2520

ccacctggca gcgtccgacg gcagcagcca ccccggatgg catgcggaga tcggggtcca 2580

tccagcagat ggagcaactc aacaggcggt atcaaaacat tcagcgaacc attgcaacag 2640

agaggtccga agaagattct ggcagccaaa gctgcgagca agccccagca ggaggaggcg 2700

gaggtggagg gagtgactca gaagccgaat cttcccagtc cagcttagat ctaaggagag 2760

aggggtcact ttctccagtg aactcacaaa aaatcacctt gctgctgcag tccccagcgg 2820

tcaagttcat caccaacccc gagttcttca ctgtgctaca tgccaattat agtgcctacc 2880

gagtcttcac cagtagcacc tgcttaaagc acatgattct gaaagtccga cgggatgctc 2940
gcaattttga acgctaccag cacaaccggg acttggtgaa tttcatcaac atgttcgcag 3000
acactcggct ggaactgccc cggggctggg agatcaaaac ggaccagcag ggaaagtctt 3060
ttttcgtgga ccacaacagt cgagctacca ctttcattga cccccgaatc cctcttcaga 3120
acggtcgtct tcccaatcat ctaactcacc gacagcacct ccagaggctc cgaagttaca 3180
gcgctggaga ggcctcagaa gtttctagaa acagaggagc ctctttactg gccaggccag 3240
gacacagctt agtagctgct attcgaagcc aacatcaaca tgagtcattg ccactggcat 3300
ataatgacaa gattgtggca tttcttcgcc agccaaacat ttttgaaatg ctgcaagagc 3360
gtcagccaag cttagcaaga aaccacacac tcagggagaa aatccattac attcggactg 3420
agggtaatca cgggcttgag aagttgtcct gtgatgcgga tctggtcatt ttgctgagtc 3480
tctttgaaga agagattatg tcctacgtcc ccctgcaggc tgccttccac cctgggtata 3540
gcttctctcc ccgctgttca ccctgttctt cacctcagaa ctccccaggt ttacagagag 3600
ccagtgcaag agccccttcc ccctaccgaa gagactttga ggccaagctc cgcaatttct 3660
acagaaaact ggaagccaaa ggatttggtc agggtccggg gaaaattaag ctcattattc 3720
gccgggatca tttgttggag ggaaccttca atcaggtgat ggcctattcg cggaaagagc 3780
tccagcgaaa caagctctac gtcacctttg ttggagagga gggcctggac tacagtggcc 3840
cctcgcggga gttcttcttc cttctgtctc aggagctctt caacccttac tatggactct 3900
ttgagtactc ggcaaatgat acttacacgg tgcagatcag ccccatgtcc gcatttgtag 3960
aaaaccatct tgagtggttc aggtttagcg gtcgcatcct gggtctggct ctgatccatc 4020
agtaccttct tgacgctttc ttcacgaggc ccttctacaa ggcactcctg agactgccct 4080
gtgatttgag tgacctggaa tatttggatg aggaattcca ccagagtttg cagtggatga 4140
aggacaacaa catcacagac atcttagacc tcactttcac tgttaatgaa gaggtttttg 4200
gacaggtcac ggaaagggag ttgaagtctg gaggagccaa cacacaggtg acggagaaaa 4260
acaagaagga gtacatcgag cgcatggtga agtggcgggt ggagcgcggc gtggtacagc 4320
agaccgaggc gctggtgcgc ggcttctacg aggttgtaga ctcgaggctg gtgtccgtgt 4380
ttgatgccag ggagctggag ctggtgatag ctggcaccgc ggaaatcgac ctaaatgact 4440

ggcggaataa cactgagtac cggggaggtt accacgatgg gcatcttgtg atccgctggt 4500
tctgggctgc ggtggagcgc ttcaataatg agcagaggct gagattactg cagtttgtca 4560
cgggaacatc cagcgtgccc tacgaaggct tcgcagccct ccgtgggagc aatgggcttc 4620
ggcgcttctg catagagaaa tgggggaaaa ttacttctct ccccagggca cacacatgct 4680
tcaaccgact ggatcttcca ccgtatccct cgtactccat gttgtatgaa aagctgttaa 4740
cagcagtaga ggaaaccagc acctttggac ttgagtgagg acatggaacc tcgcctgaca 4800
ttttcctggc cagtgacatc acccttcctg ggatgatccc cttttccctt tcccttaatc 4860
aactctcctt tgattttggt attccatgat tttatttttc aaaccaaatc aggattgaca 4920
aaagctgtgc atgaagaact gccttcttct aagatctaac cttcaggctt ctctcctctg 4980
ttttcaatga actgctagcc tgtatgcaat attaaaaaac agctgtctca aggtctgtgt 5040
atatctccac atacctccat tactaacaat gaaatatgaa tgcaagttaa gctacacttg 5100
accaaatggt aataaatgtt tacttccatt tctatcattg aagggaaaat gtgagcatta 5160
agcactccag gctttcatat gcccatgtct tctgagcaga gccaccattt tttataattt 5220
ctaataacca actccagaac taggagctga tcaactcttt gttttcctct ccatctactt 5280
ttccctgtgc ataatatcca tccaaaggac aacagtggca aagctgaaat ttttatacat 5340
tcaactcatg attcacatgt ggcatcagtc ccatcagccg gaactagcct agacatacgg 5400
tgcaaatatg acacttctaa cgattaacaa cagcaagaaa acacctgctg ctgatgcaat 5460
gcaatgcatc ccaatggttg tggggattgt gggctcaact caagagaagt ttaggagggg 5520
gagcatccct agtgaatact cacaccacaa gaaggacaaa cttgtgcaca tgtccaagaa 5580
agaaagcttc ttgattgagg tagcatgaag gatgaggctt cagcccccat tgtcttatgt 5640
agaatgtggc aatgccaact ggagaaaggg aagaaggaca tattaccttg gtttgaatcc 5700
ctgagttctg tactgttctg ttttgtttag tctagccaca gttcttcaca aaggaaaaaa 5760
aaatgtgtag atgataccat gacttttgtt aaagccatga cttttgtttg cttggcagac 5820
aaaccctttt tttaaaactt tgatattttt ttttcacatt tttttccttt ttcctttctt 5880
aatcatggag ttcaagttcc tttgcattcg attgtccatc gggaccacac taggaagctg 5940
cagagagtga tggtgcttgt tagggatcaa gggcaacata gtacttctcc ttcacccata 6000

gtaatcctcc tggggcagaa acataacacc ccaaaggcac gttgatttgt atcaaaataa 6060

atatccagtt tcttttagca ttcagtgaaa acatatctca gaaaacttca tgttgtcaga 6120

aaaacagctg caggctccaa agacagccta acctctcaac tacatttgaa ataaacccaa 6180

ccataatggt aaaaaaaaaa 6200

<210> 3

<211> 18

<212> DNA

<213> Artificial Sequence

<220>

<223> Description of Artificial Sequence:synthetic

polynucleotide

<400> 3

ctgcaccaac aatatccc 18

<210> 4

<211> 18

<212> DNA

<213> Artificial Sequence

<220>

<223> Description of Artificial Sequence:synthetic

polynucleotide

<400> 4
gtagagacag ggtttcac                                                    7